(19) **Europäisches Patentamt**
**European Patent Office**
**Office européen des brevets**

(11) **EP 2 387 359 B1**

(12) **FASCICULE DE BREVET EUROPEEN**

(45) Date de publication et mention
de la délivrance du brevet:
**11.07.2012 Bulletin 2012/28**

(51) Int Cl.:
**A61B 6/03** *(2006.01)*    **H01J 35/14** *(2006.01)*

(21) Numéro de dépôt: **09799072.5**

(22) Date de dépôt: **10.12.2009**

(86) Numéro de dépôt international:
**PCT/EP2009/066818**

(87) Numéro de publication internationale:
**WO 2010/081598 (22.07.2010 Gazette 2010/29)**

(54) **DISPOSITIF D'IMAGERIE A RAYONS X OU INFRAROUGES COMPRENANT UN LIMITEUR DE DOSE A VITESSE DE TRANSLATION CONTROLEE**

**RÖNTGEN- ODER INFRAROT-BILDGEBUNGSGERÄT MIT EINEM DOSISBEGRENZER UND KONTROLLIERTER ÜBERSETZUNGSGESCHWINDIGKEIT**

**X-RAY OR INFRARED IMAGING DEVICE COMPRISING A DOSE LIMITER, WITH CONTROLLED TRANSLATION SPEED**

(84) Etats contractants désignés:
**AT BE BG CH CY CZ DE DK EE ES FI FR GB GR HR HU IE IS IT LI LT LU LV MC MK MT NL NO PL PT RO SE SI SK SM TR**

(30) Priorité: **13.01.2009  FR 0900118**
              **27.04.2009  FR 0902018**

(43) Date de publication de la demande:
**23.11.2011  Bulletin 2011/47**

(73) Titulaire: **Beyrard, Norbert**
**01220 Divonne-Les-Bains (FR)**

(72) Inventeurs:
• **HERESON, Franck**
  **F-38600 Fontaine (FR)**
• **BEYRARD, Norbert**
  **F-01220 Divonne-Les-bains (FR)**

(74) Mandataire: **Gaglione, Renaud**
**240, rue du Quart**
**01710 Thoiry (FR)**

(56) Documents cités:
**DE-A1-102006 005 619     FR-A1- 2 844 916**
**FR-A1- 2 888 374          US-A- 5 857 008**
**US-A1- 2003 063 703      US-A1- 2008 118 023**

**Description**

L'invention se rapporte à un dispositif d'imagerie X ou infrarouge, du type radiographie ou scanographie, comprenant plus particulièrement :

- un support pour recevoir un corps à examiner,

- une source alimentant un foyer émettant un faisceau de rayons X ou lumineux traversant une fente d'un limiteur de dose commandé en translation pour balayer le corps à examiner,

- un détecteur commandé en translation pour être irradié ou illuminé par le faisceau balayant le corps à examiner et détecter une intensité atténuée en fonction d'une traversée des rayons X ou lumineux à travers le corps à examiner,

- un convertisseur analogique-numérique pour convertir les intensités détectées en données pour déterminer une atténuation par le corps à examiner des rayons X ou lumineux, et

- un ordinateur programmé pour traiter les données provenant de la conversion des intensités détectées pour aboutir à une image exprimant l'atténuation des rayons X ou lumineux par le corps examiné.

Un dispositif de ce type est connu notamment de la demande de brevet FR-A-2 888 374 déposée par le demandeur. Le détecteur se présente sous la forme d'une barrette de détection et le limiteur de dose est commandé en translation à la même vitesse et dans le même sens que le détecteur pour réduire l'exposition du corps à examiner à celle juste nécessaire à l'irradiation ou l'illumination de la barrette. La source étant fixe, le déplacement synchrone du limiteur de dose et du détecteur tend à désaligner ces trois éléments. L'effet du désalignement reste toutefois acceptable pour les rayons émis par la source avec un faible angle de divergence par rapport à la direction axiale du faisceau et la source peut être maintenue en position fixe pour un balayage d'une largeur typique. Pour un balayage plus important, par exemple du double de la largeur typique, l'effet du désalignement conduit à effectuer successivement deux balayages à partir de deux positions fixes de la source.

Le but de l'invention est de modifier un dispositif selon le type indiqué ci-dessous pour augmenter la largeur de balayage pour une position donnée de la source en vue d'obtenir des images en radiographie ou scanographie de grande largeur, typiquement de l'ordre de 50 cm, tout en réduisant l'exposition aux rayons X ou lumineux à celle juste nécessaire à l'irradiation ou l'illumination du détecteur.

A cet effet, l'invention a pour objet un dispositif conforme à celui rappelé en introduction, caractérisé en ce que le limiteur de dose est commandé en translation à une vitesse proportionnelle à celle du détecteur, contrôlée par le rapport des distances entre d'une part le foyer et le détecteur et d'autre part, le foyer et le limiteur de dose.

Par un tel contrôle des vitesses du limiteur de dose et du détecteur, on maintient un alignement entre le foyer, la fente du limiteur de dose et le détecteur. Sans déplacer la source, on peut ainsi balayer le corps à examiner sur une largeur importante en utilisant non seulement les rayons faiblement divergents mais également les rayons plus divergents, à l'intérieur d'un cône de divergence choisi en fonction des distances entre d'une part le foyer et le limiteur de dose et d'autre part entre le foyer et le détecteur. L'alignement entre le foyer, la fente du limiteur de dose et le détecteur permet de balayer le corps à examiner tout en réduisant la dose d'irradiation ou d'illumination à celle juste nécessaire à l'irradiation ou l'illumination du détecteur.

Selon une variante de réalisation de l'invention, le faisceau de rayons X ou lumineux est un faisceau secondaire, issu d'un foyer virtuel formé par un faisceau primaire, émis par un foyer réel alimenté par la source et réfléchi par un réflecteur à double réflexion disposé entre la source et le limiteur de dose. Le limiteur de dose est déplacé en translation dans le même sens de déplacement que celui du détecteur, lorsqu'il est disposé entre le foyer virtuel et le corps à examiner. Il est déplacé dans le sens opposé à celui du détecteur lorsqu'il est disposé entre le réflecteur à double réflexion et le foyer virtuel.

L'invention s'étend à un dispositif conforme à celui rappelé en introduction, caractérisé en ce que le limiteur de dose est commandé en translation à la même vitesse que celle du détecteur devant un réflecteur, irradiant ou illuminant le corps à examiner en réfléchissant les rayons émis par la source suivant des nappes parallèles.

On peut ainsi balayer le corps à examiner sur une largeur importante correspondant à une section de sortie du réflecteur sans déplacer la source mais en utilisant les rayons réfléchis suivant les nappes parallèles. Le contrôle du déplacement synchrone de la fente du limiteur de dose et du détecteur permet là encore de réduire la dose d'irradiation ou d'illumination à celle juste nécessaire à l'irradiation ou l'illumination du détecteur.

L'invention permet avantageusement d'obtenir des images de grande largeur à haute définition, d'une façon bien plus économique, par comparaison à un dispositif dans lequel une plaque de détection devrait couvrir toute la largeur d'exploration. Il faut rappeler que pour une image ayant une définition égale à 81 $\mu$m, le nombre de points à

enregistrer par cm$^2$ vaut 15241. Si l'image a une largeur de 50 cm et une hauteur de 46 cm, elle nécessite l'enregistrement de 35 millions de points. Pour une définition de 27 $\mu$m, il faut enregistrer 137174 points par cm$^2$, soit 315 millions de points pour l'image 50 cm x 46 cm. Une plaque de détection capable d'effectuer de tels enregistrements aurait un coût de fabrication prohibitif.

De préférence, le limiteur de dose est commandé en déplacement, identiquement ou proportionnellement, à la vitesse du détecteur égale au terme $\dfrac{U}{N\tau}$ où U est la largeur utile du détecteur irradiée ou illuminée par le faisceau à travers la fente du limiteur de dose, N est le nombre de lignes à K photodiodes du détecteur et $\tau$ est le temps de transfert de charge des photodiodes d'une ligne à la ligne adjacente, cumulativement de la première ligne $l_1$ à la dernière ligne $l_N$ du détecteur, ce dernier étant déplacé en sens contraire au sens du transfert de charge, soit en translation de la gauche vers la droite si la dernière ligne $l_N$ marque un bord gauche du détecteur et la première ligne $l_1$, un bord droit du détecteur.

Par comparaison avec un détecteur ne possédant qu'une seule ligne de K photodiodes, le détecteur à transfert de charge indiqué ci-dessus permet d'intégrer sur la dernière ligne $l_N$ l'intensité détectée en un point du détecteur pendant la durée $N\tau$. On peut ainsi diminuer la puissance de la source à raison du temps d'intégration $N\tau$, à même dose d'irradiation ou d'illumination reçue par le corps à examiner. De surcroît, la largeur utile U est N fois plus importante que celle d'un détecteur à une seule ligne, à même définition de détection, c'est-à-dire même largeur de ligne. En commandant en déplacement le limiteur de dose à une vitesse U identique ou proportionnelle à celle du détecteur, égale à la vitesse $\dfrac{U}{N\tau}$, on diminue ainsi la surexposition locale du détecteur, définie par la largeur de la projection de la fente du limiteur de dose sur le détecteur, rapportée à la largeur utile du détecteur.

D'autres avantages de l'invention apparaîtront à la lumière de la description des modes de réalisation illustrés par les dessins.

La figure 1 est une vue de dessus d'un dispositif selon un premier mode de réalisation de l'invention.

La figure 2 est une vue de dessus d'une variante à foyer virtuel du premier mode de réalisation de l'invention, dans laquelle le limiteur de dose est disposé en aval d'un foyer virtuel formé par un réflecteur à double réflexion à un seul axe.

La figure 3 est une vue de dessus d'une variante à foyer virtuel du premier mode de réalisation de l'invention, dans laquelle le limiteur de dose est disposé en amont du foyer virtuel formé par un réflecteur à double réflexion à un seul axe.

La figure 4 est une vue de dessus d'une variante à foyer virtuel du premier mode de réalisation de l'invention, dans laquelle le limiteur de dose est disposé en aval du foyer virtuel formé par un réflecteur à double réflexion à deux axes.

La figure 5 est une vue de dessus d'une variante à foyer virtuel du premier mode de réalisation de l'invention, dans laquelle le limiteur de dose est disposé en amont du foyer virtuel formé par un réflecteur à double réflexion à deux axes.

La figure 6 est une vue de dessus d'un dispositif selon un deuxième mode de réalisation de l'invention, pour une première position de balayage.

La figure 7 est une vue de dessus d'un dispositif selon le deuxième mode de réalisation de l'invention, pour une deuxième position de balayage.

La figure 8 est une vue de face du limiteur de dose des dispositifs illustrés par les figures 1 à 5.

La figure 9 est une vue de face d'une exécution particulière du limiteur de dose illustré par la figure 8.

La figure 10 est une vue de face du détecteur des dispositifs illustrés par les figures 1 à 5.

La figure 11 montre un segment isolé du détecteur illustré par la figure 10, à différents instants du déplacement en translation du détecteur.

La figure 12 montre schématiquement la structure du détecteur ainsi que les éléments de la chaîne d'acquisition du dispositif illustré par les figures 1 à 5.

La figure 13 est une vue de dessus montrant un système de centrage des prises de vue.

La figure 14 illustre la mise en oeuvre du centrage dans une première prise de vue de référence.

La figure 15 illustre la mise en oeuvre du centrage dans une deuxième prise de vue de référence.

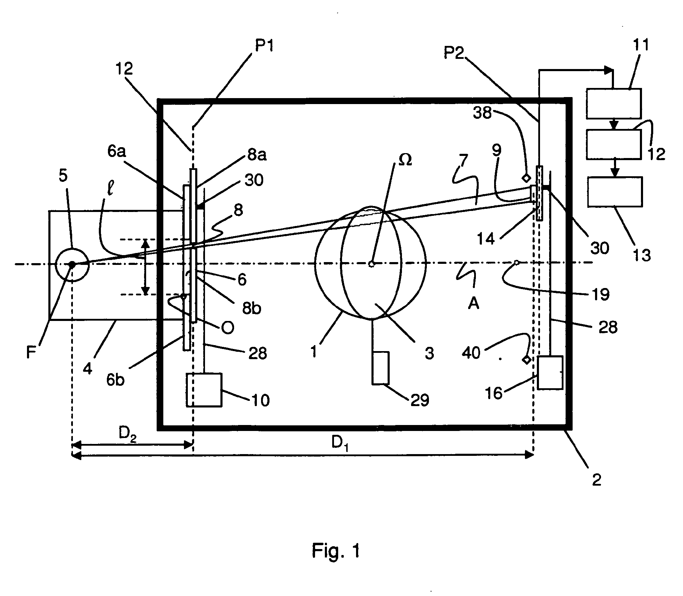

Figure 1, un premier mode de réalisation d'un dispositif d'imagerie X ou infrarouge, conçu pour la radiographie ou la scanographie, comprend dans une cabine 2 :

- un support 1 pour recevoir un corps à examiner 3,

- une source 5 alimentant un foyer F émettant un faisceau 7 de rayons X ou lumineux traversant une fente 8 d'un limiteur de dose 8,8a,8b commandé en translation balayer le corps à examiner 3,

- un détecteur 9 commandé en translation pour être irradié ou illuminé par le faisceau 7 balayant le corps à examiner 3 et détecter une intensité atténuée en fonction d'une traversée des rayons X ou lumineux à travers le corps à examiner 3,

- un convertisseur 11 analogique-numérique pour convertir les intensités détectées en données pour déterminer une atténuation par le corps à examiner 3 des rayons X ou lumineux, et

- un ordinateur 13 programmé pour traiter les données provenant de la conversion des intensités détectées pour aboutir à une image exprimant l'atténuation des rayons X ou lumineux par le corps examiné 3.

Le support 1 peut tourner autour d'un axe de rotation $\Omega$, définissant conventionnellement le sens de la hauteur.
La source 5 est fixée dans une cage 4 de forme parallélépipédique présentant une section de sortie 6 délimitée par deux plaques 6a,6b espacées l'une de l'autre d'une largeur $\ell$. A noter que ces deux plaques peuvent être coulissantes par rapport à la cage 4 pour faire varier la largeur $\ell$ de la section de sortie 6. Elle émet le faisceau à partir du foyer F placé dans un tube à rayons X. Les positions respectives du foyer F et des plaques 6a,6b déterminent une direction axiale A de propagation du faisceau hors de la cage parallélépipédique. La largeur $\ell$ de la section de sortie 6 détermine la divergence du faisceau autour de la direction axiale A. Le limiteur de dose 8a,8b comprend deux plaques 8a,8b disposées de façon coplanaire dans un plan P1 perpendiculaire à la direction axiale A du faisceau. Les deux plaques 8a,8b sont espacées l'une de l'autre pour ménager une fente 8 de largeur e. Le limiteur de dose est commandé en translation dans le plan P1 par un vérin 10 le long d'un rail 12, de préférence un rail à billes pour limiter les frottements. La tige mobile 28 du vérin est fixée à la plaque 8a par une attache 30. La fente 8 est ainsi déplacée devant la section de sortie 6 de la cage parallélépipédique 4 pour que le faisceau balaye le corps à examiner 3. Les deux plaques utilisées sont par exemple en plomb. Elles ont une largeur de 16 cm, une hauteur de 10 cm et une épaisseur de 2 mm. Le détecteur 9 est fixé à un support 14 commandé par un vérin 16 le long d'un rail, en translation dans un plan P2 perpendiculaire à la direction axiale A du faisceau. La tige mobile 28 du vérin est fixée au support 14 par une attache 30. A noter que le vérin 16 peut être suffisamment robuste pour déplacer le détecteur 9 sans participation d'un rail, ceci pour limiter les frottements et mieux contrôler la vitesse de déplacement du détecteur 9 par rapport à celle du limiteur de dose 8a,8b.
Selon l'invention, le limiteur de dose 8,8a,8b est commandé en translation à une vitesse proportionnelle à celle du détecteur 9, contrôlée par le rapport des distances $\dfrac{D_2}{D_1}$ entre d'une part la source 5 et le détecteur 9 $D_1$ et d'autre part, la source 5 et le limiteur de dose 8,8a,8b $D_2$.
Un logiciel est chargé dans l'ordinateur 13 pour commander en translation les deux vérins 10,16 déplaçant le limiteur de dose 8,8a,8b et le détecteur 9. A titre d'exemple, la vitesse du limiteur de dose 8,8a,8b est égale au tiers de celle du détecteur 9 lorsque la distance entre la source 5 et le détecteur 9 vaut 180 cm et la distance entre la source 5 et le limiteur de dose, 60 cm. Si le détecteur est déplacé sur une course de 50 cm en vue d'obtenir une image de même largeur, le limiteur de dose est déplacé dans le même temps sur une course de 16,66 cm, dans le même sens de déplacement que le détecteur. La synchronisation entre le limiteur de dose et le détecteur s'effectue indépendamment de la source, qui reste fixe. Cet agencement permet de s'affranchir de l'inertie de la source si cette dernière devait être entraînée en déplacement par le limiteur de dose. En effet, tout dispositif qui nécessiterait la rotation du tube à rayons X présente la difficulté d'avoir à régler pas à pas un telle rotation sur un tube dont le poids peut atteindre 35 kg et qui au surplus entraîne la nécessité de déplacer aussi un câble d'alimentation haute tension et un tube de refroidissement véhiculant par exemple de l'huile. On peut rappeler que l'agencement prévu par l'invention permet de modifier sans difficulté les distances du foyer au détecteur et du foyer au limiteur de dose. Il suffira de régler en conséquence les vitesses de translation de ces deux éléments.

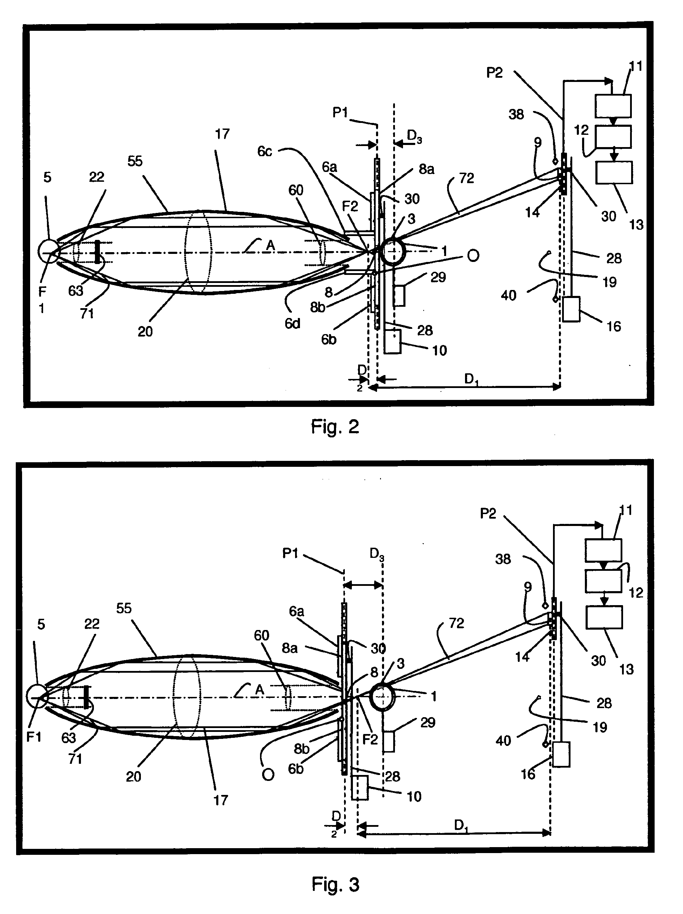

Les figures 2 à 5 illustrent différentes variantes à foyer virtuel du premier mode de réalisation de l'invention. Dans ces variantes, le faisceau de rayons X ou lumineux est un faisceau secondaire 72, issu d'un foyer virtuel F2 formé par un faisceau primaire 71, émis par un foyer réel F1 alimenté par la source 5 et réfléchi par un réflecteur à double réflexion 55 disposé entre la source 5 et le limiteur de dose 8,8a,8b.

Les figures 2 et 3 illustrent plus particulièrement un réflecteur à double réflexion 55 dont la section d'entrée 22 et la section de sortie 60 sont alignées suivant la direction axiale A du réflecteur. De préférence, ce réflecteur est un paraboloïde de révolution. Le foyer réel F1 alimenté par la source 5 se confond avec le foyer géométrique d'entrée du paraboloïde. Les rayons X ou lumineux du faisceau primaire 71 émis par le foyer réel F1 subissent une première réflexion alignant leur propagation parallèlement à la direction axiale A du réflecteur puis une deuxième réflexion modifiant leur propagation pour converger au foyer virtuel F2 confondu avec le foyer géométrique de sortie du paraboloïde. Les rayons X ou lumineux issus du foyer virtuel F2 forment le faisceau secondaire 72. Un obturateur 63 en forme de disque est disposé en aval de la section d'entrée 22 du réflecteur à double réflexion 55 pour stopper la propagation des rayons directs issus du foyer réel F1. A noter que le réflecteur 55 peut comprendre deux paraboloïdes disposés tête-bêche et déplaçables en translation suivant la direction axiale A pour faire varier la position du foyer virtuel F2 le long de cette direction axiale: Ces deux paraboloïdes peuvent être de deux tailles différentes.

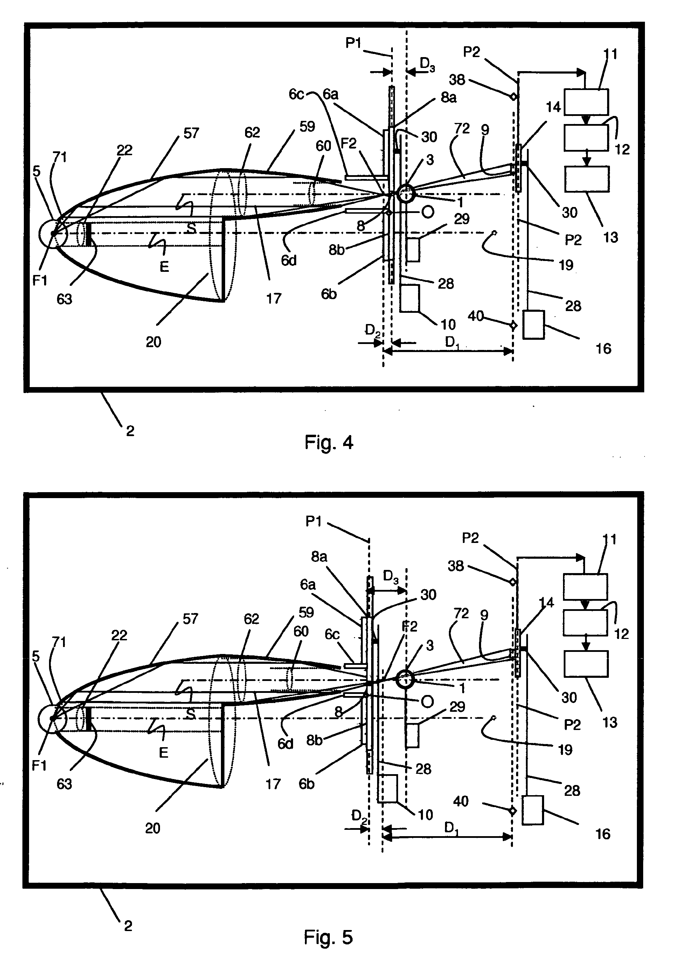

Les figures 4 et 5 illustrent plus particulièrement un réflecteur à double réflexion 55 dont la section d'entrée 22 est alignée suivant une première direction axiale E du réflecteur et la section de sortie 60, suivant une deuxième direction axiale S du réflecteur, parallèle mais non co-axiale avec la première direction axiale E. De préférence, ce réflecteur comprend deux paraboloïdes de révolution 57 et 59, disposés tête-bêche. Le foyer réel F1 alimenté par la source 5 se confond avec le foyer géométrique du paraboloïde d'entrée 57. Les rayons X ou lumineux du faisceau primaire 71 émis par le foyer réel F1 subissent une première réflexion dans le paraboloïde d'entrée 57, alignant leur propagation parallèlement à la direction axiale E. Un obturateur 63 en forme de disque est disposé en aval de la section d'entrée 22 du paraboloïde d'entrée 57 pour stopper la propagation des rayons directs issus du foyer réel F1. La grande section 62 du paraboloïde de sortie 59 est de préférence inscrite dans la région annulaire de la grande section 20 du paraboloïde d'entrée 57 traversée par les rayons réfléchis parallèlement à la direction axiale E. Ces derniers subissent une deuxième réflexion dans le paraboloïde de sortie 59, modifiant leur propagation pour converger vers le foyer virtuel F2 confondu avec le foyer géométrique de ce paraboloïde 59 appartenant à la direction axiale S. Les rayons X ou lumineux issus du foyer virtuel F2 forment le faisceau secondaire 72.

Figure 2 ou 4, le limiteur de dose 8,8a,8b est disposé en aval du foyer virtuel F2, soit encore entre ce foyer virtuel et le corps à examiner 3 et est commandé en translation dans le même sens de déplacement que le détecteur et à une vitesse proportionnelle à ce dernier, contrôlée par le rapport des distance entre d'une part le foyer virtuel F2 et le détecteur, soit D1, et le foyer virtuel F2 et le limiteur de dose 8,8a,8b d'autre part, soit D2. Figure 3 ou 5, le limiteur de dose 8,8a,8b est disposé en amont du foyer virtuel F2, soit encore entre ce foyer virtuel et le réflecteur à double réflexion 55 et est commandé en translation dans un sens de déplacement opposé à celui du détecteur et à une vitesse proportionnelle à ce dernier, contrôlée par le rapport des distance entre d'une part le foyer virtuel f2 et le détecteur, soit D1, et le foyer virtuel F2 et le limiteur de dose 8,8a,8b d'autre part, soit D2.

Les variantes illustrées par les figures 4 et 5 permettent avantageusement de disposer plusieurs réflecteurs de sortie 59 en aval du réflecteur d'entrée 57. Ainsi, la même source 5 à rayons X ou lumineux est utilisée pour explorer plusieurs corps à examiner 3 simultanément. A chaque réflecteur de sortie 59 un triplet de distances D1,D2,D3 peut être associé en réglant la position du foyer virtuel F2 ou là position du support 1 le long de la direction axiale S de chacun. Le réglage de position du foyer virtuel F2 s'obtient par exemple par une translation plus ou moins importante d'un réflecteur de sortie 59 par rapport au réflecteur d'entrée commun 57, parallèlement à la direction axiale E. Ainsi, pour une position donnée du plan de déplacement P2 du détecteur 9, différentes positions du foyer virtuel F2 ou du support 1 permettent d'obtenir différents grossissements. A noter que différentes positions du support 1 permettent, pour deux positions données du foyer virtuel F2 et du plan de déplacement du détecteur 9, d'obtenir différents grossissements avec une même cinématique entre le limiteur de dose 8,8a,8b et le détecteur 9, les distances D1 et D2 étant par hypothèse constantes, seule la distance D3 étant affectée par le réglage de la position du support 1. A titre d'exemple, on donne les valeurs numériques suivantes: le réflecteur d'entrée 57 est un paraboloïde de paramètre $\rho = 2$ ayant 60 cm de longueur et une grande section 20 égale à 31 cm de diamètre; le réflecteur de sortie 59 est un paraboloïde de paramètre ρ = 0,1 ayant 30 cm de longueur et une grande section 62 égale à 4,9 cm de diamètre.

Les différentes variantes à foyer virtuel du premier mode de réalisation de l'invention permettent de balayer le corps à examiner 3 avec le faisceau secondaire 72 tout en limitant l'exposition aux rayons X ou lumineux à celle juste nécessaire à l'irradiation ou l'illumination du détecteur. Ces variantes sont particulièrement bien adaptées à l'exploration de corps de petite taille, de quelques centimètres, comme dans le cas de petits animaux de laboratoire, ou à quelques millimètres voire quelques micromètres, comme dans le cas d'un tissu de cellules humaines, animales ou végétales. Le corps à examiner 3 peut en effet être fortement rapproché du foyer virtuel F2 pour minimiser la distance D3 les séparant, par rapport à la distance D1 existant entre le foyer virtuel F2 et le détecteur 9. Ainsi, l'image du tissu cellulaire projetée sur le détecteur 9 est agrandie dans le rapport des distances D1 et D3.

En application des variantes illustrées par les figures 2 et 4, un amas de cellules en tant que corps à examiner 3 étant disposé dans une éprouvette servant de support 1, à une distance D3 égale à 4 mm du foyer virtuel F2, on obtient un grossissement égal à 250 en disposant le détecteur 9 à une distance D1 égale à 1 m du foyer virtuel F2. Si la résolution du détecteur 9 est égale à 25 $\mu$m, on obtient une résolution d'image du tissu cellulaire égale à 100 angstrôms. Les variantes illustrées par les figures 3 et 5 permettent de rapprocher encore l'éprouvette du foyer virtuel F2, par exemple à une distance D3 égale à 1 mm, étant rappelé que le limiteur de dose 8,8a,8b est disposé en amont du foyer virtuel F2. On obtient ainsi un grossissement égal à 1000 en disposant le détecteur 9 à la distance D1 égale à 1 m et une résolution d'image du tissu cellulaire égale à 25 angströms. Dans ces différents cas de figure, on déplace le limiteur de dose 8,8a,8b en translation dans le plan P1 à une vitesse égale à 405 $\mu$m/s lorsqu'il est disposé à une distance D2 du foyer virtuel F2 égale à 5 mm et que le détecteur 9 est déplacé dans le plan P2 à une vitesse égale à 8,1 cm/s. La fente 8 du limiteur de dose est réduite à une largeur égale à 50 $\mu$m. Le vérin 10 de commande en déplacement du limiteur de dose est ici remplacé par une crémaillère à actuateur piézoélectrique, dont un exemple est donné par la demande de brevet EP-A-1 058 322 et le brevet US 6 429 572 déposés par le demandeur.

Les différentes variantes à foyer virtuel du premier mode de réalisation de l'invention décrites ci-dessous s'appliquent à l'imagerie de tout corps, vivant ou inanimé. Dans le cas due corps vivants, on peut encore réduire l'exposition aux rayons X en disposant autour du corps des feuilles métalliques, par exemple en aluminium ou en fer, pour absorber une partie de la dose émise, qui peut être importante à proximité du foyer virtuel.

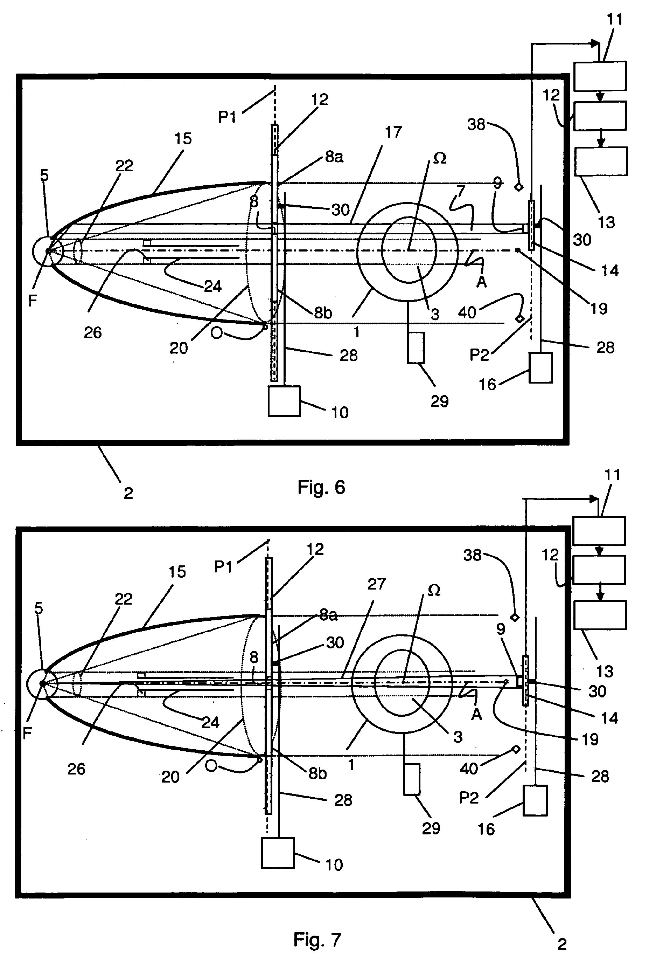

Les Figures 6 et 7 illustrent un deuxième mode de réalisation de l'invention. Dans ces figures, les éléments en commun avec le premier mode de réalisation de l'invention portent les mêmes référence et sont considérés comme décrites par renvoi à la figure 1.

Un réflecteur 15 est disposé en regard du foyer F de la source 5. En incidence rasante, les rayons X émis par la source 5 se réfléchissent totalement ou partiellement sur les parois du réflecteur 15. On détermine l'angle θ en dessous duquel la réflexion est totale par la formule suivante :

$$\theta < \lambda / (5{*}10^{-8})$$

Les longueurs d'onde λ sont liées à la tension d'alimentation V par la formule suivante :

$$V\,(kV) = 12.38 / \lambda\,(Ang)$$

Le calcul conduit rapidement aux résultats suivants :

- pour 125 kV        θ = 1,12°

- pour 70 kV        θ = 2°

- pour 35 kV        θ = 4°

Le réflecteur 15 est de préférence un paraboloïde de révolution pour paralléliser les rayons réfléchis en un faisceau cylindrique. On rappelle qu'une parabole est définie par une fonction :

$$y^2 = 2 {*} \rho {*} x$$

Le paramètre ρ détermine la section de sortie 20 du paraboloïde de révolution. A titre d'exemple, lorsque le paramètre ρ est égal à l'unité, la section de sortie à 50 cm du foyer du paraboloïde est un cercle de rayon égal à 10 cm. On peut ainsi irradier ou illuminer le corps à examiner 3 sur un cercle de 20 cm de diamètre centré sur la projection du foyer F de la source 5 sur le détecteur 9.

Si le foyer F du tube à rayons X se confond avec le foyer de la parabole générant le paraboloïde, les rayons totalement réfléchis ou réverbérés 17, figure 6, sont parallèles à la direction axiale du faisceau A confondue avec l'axe central du paraboloïde.

Les conditions de la réflexion ou de la réverbération dépendent de l'angle d'un rayon incident avec la tangente à la parabole au point d'incidence. Pour ρ = 1 et E = 75000 volts, l'angle de réflexion sur la surface du réflecteur, calculée à partir de la tangente au point considéré, entraîne une réflexion totale pour des valeurs angulaires inférieures à 2°. Le taux de réverbération mesure le rapport entre la réflexion partielle et la réflexion totale théorique. Ce taux est généralement acceptable pour des angles inférieurs à 10°.

Ce deuxième mode de réalisation se distingue du premier en ce que le limiteur de dose 8,8a,8b est commandé en translation à la même vitesse que celle du détecteur 9 devant la section de sortie 20 du réflecteur 15, irradiant ou illuminant le corps à examiner 3 en réfléchissant les rayons émis par la source 5 suivant des nappes parallèles.

Un logiciel est chargé dans l'ordinateur 13 pour commander en translation à la même vitesse les deux vérins 10,16 déplaçant le limiteur de dose 8,8a,8b et le détecteur 9. Si le détecteur est déplacé sur une course de 50 cm, le limiteur de dose est déplacé dans le même temps sur une même course.

La figure 6 montre le dispositif selon le deuxième mode de réalisation dans une position du balayage pour laquelle le détecteur 9 est irradié ou illuminé par les seuls rayons réfléchis 17 traversant la fente 8 du limiteur de dose 8,8a,8b parallèlement à la direction axiale A du faisceau 7. Dans cette position du balayage, les rayons directs, c'est-à-dire non réfléchis par le réflecteur parabolique 15, qui traversent la fente 8 du limiteur de dose 8,8a,8b sont trop désalignés par rapport à l'alignement de la fente 8 et du détecteur 9, parallèlement à la direction axiale A du faisceau, pour atteindre le détecteur 9.

La figure 7 montre le dispositif précédent dans une deuxième position du balayage pour laquelle le détecteur 9 est irradié ou illuminé par les seuls rayons directs 27 traversant la fente 8 du limiteur de dose 8,8a,8b.

La zone de transition du balayage par les rayons réfléchis au balayage par les rayons directs est déterminée par la section d'entrée 22 du réflecteur parabolique 15. On ne peut exclure que le détecteur soit irradié à la fois par des rayons réfléchis 17 et des rayons directs 27 lorsque la fente 8 entre ou sort de la zone correspondant à la section d'entrée 22 du réflecteur parabolique 15. Pour limiter cet effet, on prévoit de disposer dans le réflecteur parabolique, un tube de collimation 24 coaxial avec l'axe central du paraboloïde 15 et fixé à un disque perforé 26 coïncidant avec la section d'entrée 22 du réflecteur parabolique 15. Le tube de collimation 24 concentre les rayons directs 27 sur la fente 8 du limiteur de dose 8a,8b lorsqu'elle est déplacée devant la zone correspondant à la section d'entrée 22 du réflecteur parabolique 15. La collimation augmente l'intensité du faisceau sortant du tube de collimation 24 par la capture de rayons directs à l'entrée du tube et leur réverbération à l'intérieur du tube.

Par comparaison avec un dispositif ne comportant pas de limiteur de dose, la dose d'irradiation ou d'illumination reçue par le corps à examiner pendant le temps mis par le détecteur 9 pour effectuer la course de balayage correspondante, est divisée par le rapport des aires de la fente 8 du limiteur de dose d'une part et de la section de sortie 6 de la cage parallélépipédique 2 ou 20 du réflecteur 15.

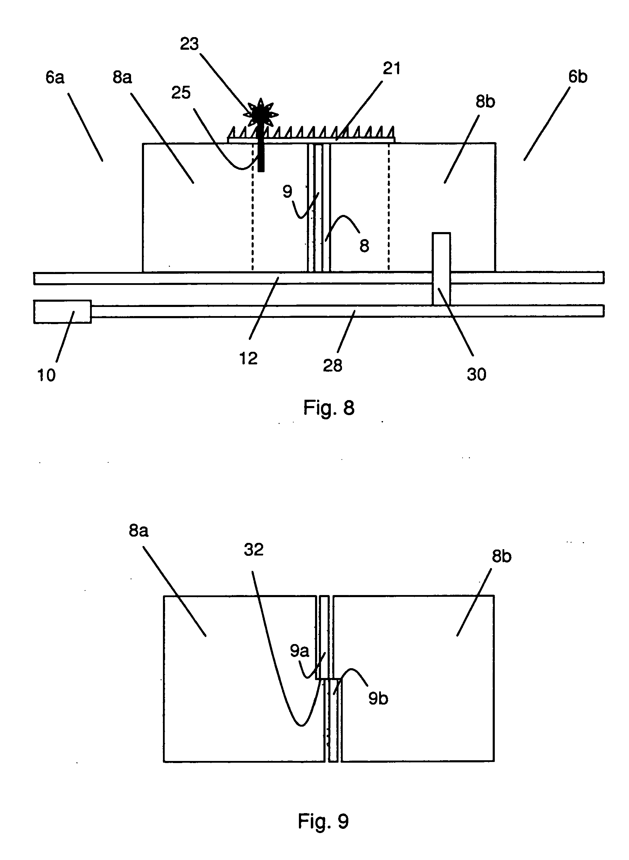

La figure 8 illustre un agencement préféré du limiteur de dose 8a,8b dans lequel les deux plaques 8a,8b sont déplaçables l'une par rapport à l'autre par un moyen de réglage de la largeur e de la fente 8. Ce moyen comprend une crémaillère 21 fixée à l'une 8b des deux plaques et une roue crantée 23 portée par un axe 25 fixé à l'autre plaque 8a pour s'engager dans la crémaillère 21. On prévoit également de régler l'ouverture de la fente 8 par l'intermédiaire d'une vis traversant deux filetages solidaires des deux plaques. Le réglage de l'ouverture de la fente 8 permet de contrôler un facteur de surexposition locale défini par la largeur de la projection de la fente 8 du limiteur de dose sur le détecteur 9, rapportée à la largeur utile du détecteur.

La figure 9 illustre un agencement préféré du détecteur 9 à deux barrettes de détection 9a,9b pour doubler la hauteur de prise de vue. Les deux barrettes 9a,9b sont de préférence décalées l'une par rapport à l'autre pour éviter la formation d'une zone neutre dans l'image due à un défaut d'ajustement en hauteur. En ce cas, les plaques du limiteur de dose 8a,8b présentent un profil de fente 32 correspondant au décalage des deux barrettes de détection pour maintenir une largeur de fente constante en face de la première 9a comme de la deuxième barrette de détection 9b. Ainsi, le facteur de surexposition locale n'est pas augmenté par le décalage des deux barrettes de détection.

Dans le premier ou le deuxième mode de réalisation de l'invention, le limiteur de dose 8,8a,8b est de préférence commandé en translation par le vérin 10 à partir d'un point de repos O pour lequel la fente 8 est en dehors de la section de sortie 6 de la cage parallélépipédique 4 ou de la section de sortie 20 du réflecteur 15 pour obstruer complètement le faisceau 7. Dans les exemples de réalisation illustré par les figures 2 à 5, le point de repos correspond à une position de la fente 8 en dehors de la section de sortie 60 du réflecteur à double réflexion 55. Si le limiteur de dose est disposé à une certaine distance de la section de sortie 60 du réflecteur à double réflexion 55, il est prévu d'encager le faisceau entre la section de sortie 60 et le limiteur de dose. Un tube 6c,6d s'étend parallèlement à la direction axiale A du faisceau et est fixé aux deux parois 6a,6b espacées de la largeur ℓ et disposées dans le plan P1. Le limiteur de dose 8a,8b est déplacé en translation dans le plan P1 au plus près des parois 6a,6b pour obstruer le faisceau lorsque la fente 8 est dans la position de repos O.

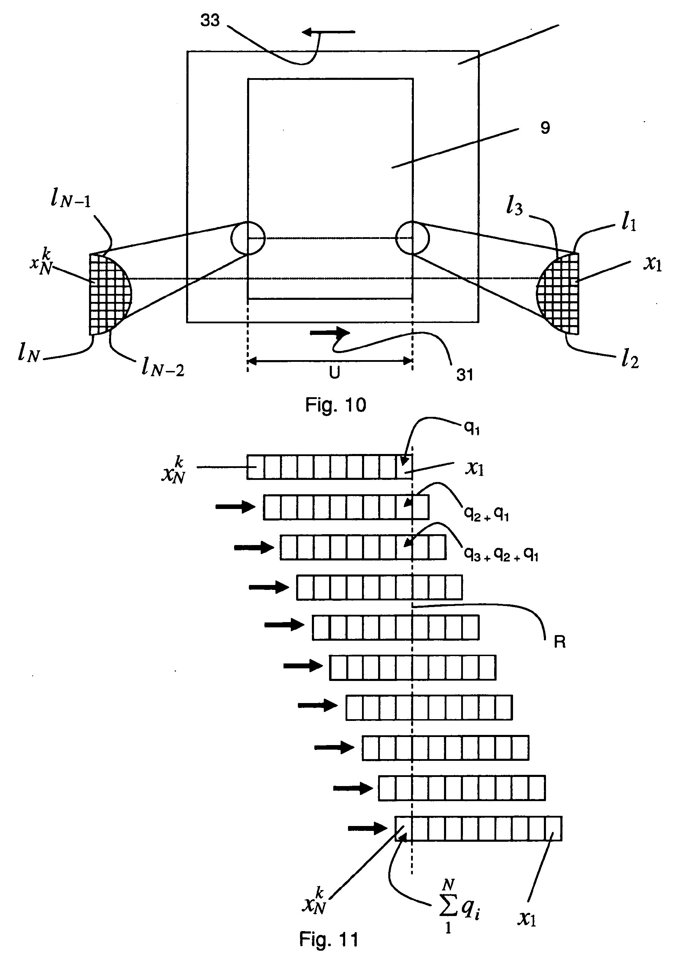

En pratique, il est prévu par exemple de débuter la translation du détecteur 9 à partir du point correspondant à la projection du point de repos O du limiteur de dose sur le plan P2, ou de débuter la translation du détecteur 9 avec un retard par rapport à la translation du limiteur de dose, correspondant au temps mis par la fente 8 pour entrer dans la section de sortie de la cage parallélépipédique 4 ou de la section de sortie 20 du réflecteur 15, à partir du point de repos O. Cet agencement permet de faire fonctionner la source 5 sans nécessiter de l'éteindre puis de la réenclencher et diminue ainsi le temps mort entre deux prises de vue. Cela revêt également une grande importance au plan économique, dans la mesure où un fonctionnement en continu de la source augmente considérablement sa durée de service. Au contraire, des allumages et des arrêts répétés entraîneraient une dégradation rapide du tube à rayons X. Les figures 10 et 11 illustrent un mode de réalisation de l'invention avec un détecteur 9 comprenant N lignes de K photodiodes $x_n^k$ chacune. Le nombre N de lignes détermine la définition du détecteur dont la largeur utile est U. La charge des photodiodes de même indice k est transférée d'une ligne à la ligne adjacente, cumulativement de la première ligne $l_1$ à la dernière ligne $l_N$ du détecteur 9, avec un temps de transfert $\tau$.

Figure 11, la charge $q_1$ acquise pendant $\tau$ par la photodiode $x_1^k$ est transférée à la photodiode $x_2^k$. Dans le même temps, cette photodiode $x_2^k$ est déplacée avec le déplacement du détecteur 9 pour occuper, dans un repère fixe R par rapport à la cabine 2, la position qu'occupait la photodiode $x_1^k$ avant le transfert. La photodiode $x_2^k$ acquiert une nouvelle charge $q_2$ pendant $\tau$ et transfère la charge cumulée $q_1 + q_2$ à la photodiode $x_3^k$. Dans le même temps, cette photodiode $x_3^k$ est déplacée par la translation du détecteur 9 pour occuper, dans un repère fixe R par rapport à la cabine 2, la position qu'occupait la photodiode $x_2^k$ avant le transfert de charge. La photodiode $x_N^k$ de même indice appartenant à la dernière ligne cumule, à l'issue des N transferts de charge, la somme des charges acquises par chaque photodiode de même indice k à chaque instant $\tau$.

Chaque photodiode de la dernière ligne $l_N$ intègre ainsi l'intensité correspondant à l'irradiation ou l'illumination du corps à examiner 3 par un même rayon X ou lumineux pendant la durée du transfert des charges de la première ligne $l_1$ à la dernière ligne $l_N$, soit pendant $N\tau$, lorsque le détecteur 9 est déplacé à la vitesse $\dfrac{U}{N\tau}$ en sens contraire au sens du transfert de charge. Sur les figures 6 et 7, le détecteur est conventionnellement déplacé de la gauche vers la droite comme indiqué par la flèche 31, alors que les charges des photodiodes de même indice k remontent, comme indiqué par la flèche 33, vers la dernière ligne $l_N$ marquant un bord gauche du détecteur 9 à partir de la première ligne $l_1$ marquant un bord droit du détecteur 9.

La dose d'irradiation du corps à examiner est proportionnelle à la puissance de la source et au temps d'exposition. Pour obtenir une image sous une même définition, par exemple 27 μm, avec une même dose d'irradiation, l'intégration pendant $N\tau$ permet de diviser par $N\tau$ la puissance de la source. Le détecteur à transfert de charge permet également de diminuer le facteur de surexposition locale défini précédemment, puisque la largeur utile du détecteur est N fois plus importante, comparée à celle d'un détecteur à une seule ligne de détection.

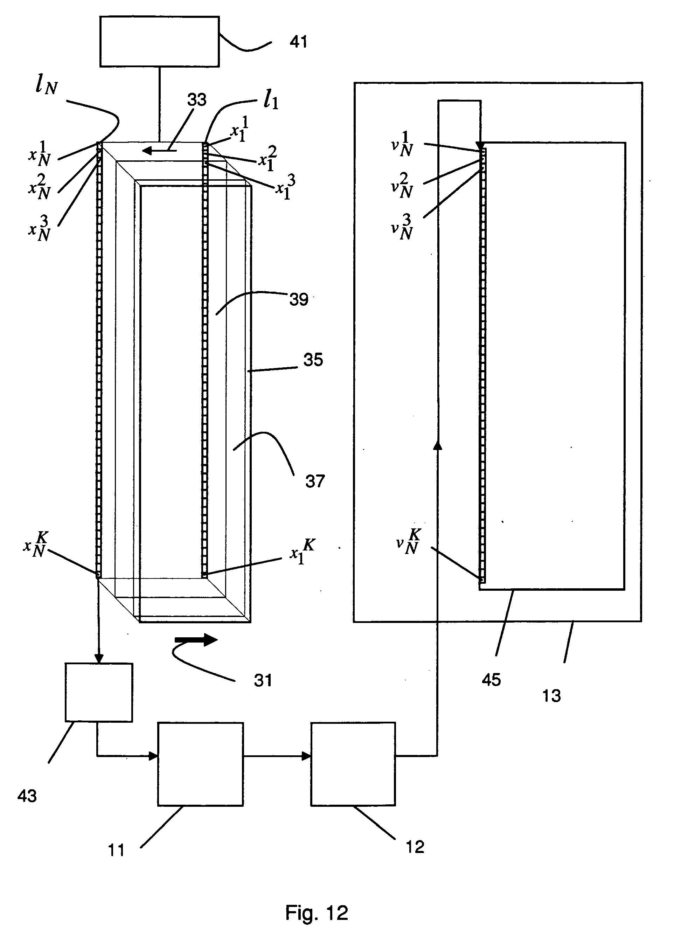

La figure 12 montre de façon schématique la structure du détecteur 9 ainsi. que les éléments de la chaîne d'acquisition.

Le détecteur 9 comprend un scintillateur 35, un réseau de fibres optiques 37 et un capteur 39 à photodiodes rangées en N lignes de chacune K photodiodes. Sous l'effet des rayons X, le scintillateur 35 émet des paquets de photons infrarouges qui sont guidés par les fibres optiques 37 jusqu'à illuminer les photodiodes. Un générateur de phase 41 est accouplé au détecteur 9 pour effectuer d'une part le transfert de charge des photodiodes $x_1^1$, $x_1^2$, $x_1^K$ de la première ligne $l_1$ aux photodiodes $x_N^1$, $x_N^2$, $x_N^K$ de la dernière ligne $l_N$, suivant le procédé décrit précédemment, et le transfert de la dernière ligne $l_N$ vers un amplificateur 43 puis vers le convertisseur analogique- numérique 11. La conversion est réalisée sur 8 ou 12 bits.

A noter que le détecteur 9 et l'amplificateur 43 peuvent être refroidis par azote à -160°C -190°C pour diminuer le bruit de fond d'origine thermique et augmenter le gain d'amplification. De même, le générateur de phase 41 est refroidi à -20°C. Le refroidissement du détecteur 9, du générateur de phase 41 et de l'amplificateur 43 augmente la sensibilité de la chaîne d'acquisition et contribue ainsi à la réduction de la dose d'irradiation.

Une interface 12 est prévue entre le convertisseur 11 analogique- numérique et l'ordinateur 13 pour transférer à une fréquence égale à l'inverse du temps $\tau$ de transfert de charge, les données $v_N^1$, $v_N^2$, $v_N^K$ issues de la conversion des charges cumulées sur chacune des photodiodes $x_N^1$, $x_N^2$, $x_N^K$ de la dernière ligne $l_N$ du détecteur 9. Les données sont rangées dans la mémoire de l'ordinateur 13 dans un tableau 45 traduisant l'image obtenue par les déplacements à vitesse contrôlée, proportionnelle ou identique, du limiteur de dose 8a,8b et du détecteur 9 pendant le balayage du corps à examiner 3.

Un détecteur conforme à celui qui vient d'être décrit est disponible sous la marque ATMEL et la référence AT71957M. Le nombre de lignes N est égal à 242 et chaque ligne possède 8520 photodiodes. Le détecteur se présente sous la forme d'une barrette de détection ayant une largeur utile U égale à 0,654 cm et une hauteur utile égale à 23 cm. Chaque ligne a une définition de 27 $\mu$m dans la largeur comme dans la hauteur. Le temps de transfert de charge $\tau$ est égal à 1 ms. Pour une course de déplacement de 50 cm, la dose d'irradiation est divisée par 76,45 si la largeur de projection de la fente 8 du limiteur de dose 8a,8b sur le détecteur 9 est égale à la largeur utile du détecteur, soit 0,654 cm. Pour une largeur de projection de 1 cm, la dose est divisée par 50 et le facteur de surexposition locale vaut 1,53.

Le limiteur de dose 8a,8b est déplacé en sens contraire à celui du transfert de charge, à la vitesse $\dfrac{U}{N\tau}$ égale à 2,7 cm/s. Le temps $N\tau$ d'exposition de toute partie du corps à examiner 3 aux rayons X ou infrarouges est limité à 242 ms. Il est possible de regrouper électroniquement les photodiodes de trois lignes adjacentes pour passer d'une définition de 27 $\mu$m à une définition de 81 $\mu$m. Le détecteur 9 est alors déplacé à la vitesse de 8,1 cm/s et le temps d'exposition de toute partie du corps à examiner diminue à 80 ms.

Le dispositif d'imagerie X ou infrarouge selon l'invention s'applique à la radiographie ou à la scanographie. Dans le cas de la radiographie, on prend généralement deux vues du corps à examiner, l'une de face et l'autre de profil. Dans le cas de la scanographie, on est amené à prendre plusieurs vues sous différents angles de rotation pour aboutir à une image des coefficients d'atténuation du corps examiné dans des plans de coupe perpendiculairement à la rotation.

Dans la suite de l'exposé, on se limitera à l'application du dispositif à la scanographie. Pour un rappel du principe à la base de cette technique d'imagerie ainsi que pour un exposé complet du procédé de scanographie par génération directe auquel il est fait référence désormais, on consultera avec intérêt la demande française FR 2 888 374 et la demande internationale WO 2007/006560 déposées par le demandeur. On pourra également consulter la demande française FR 2 871 911 et la demande internationale WO 2006/003312 déposées par le demandeur pour un procédé de scanographie par amplification matricielle et ajustement point par point.

Le dispositif est plus particulièrement défini par le fait qu'au cours de l'acquisition des données, le support 1 est commandé en rotation autour de l'axe de rotation $\Omega$, par un moteur 29 et par le fait que l'ordinateur 13 est programmé pour effectuer les étapes suivantes :

(1) construire un vecteur générateur colonne et un vecteur générateur ligne dont les n et m termes sont respectivement constitués par les données ci et $\rho_j$ provenant de la conversion des intensités détectées en un même point $X_N$ du détecteur 9 pendant la translation de ce dernier pour respectivement un premier i et un deuxième j angle de rotation, de préférence différents entre eux de 90 degrés, du support 1 autour de l'axe de rotation $\Omega$, les données correspondant à un quadrillage en n x m zones élémentaires d'un plan de coupe du corps à examiner 3, perpendiculaire à l'axe de rotation $\Omega$ et contenant le point considéré $X_N$ du détecteur 9,

(2) construire une matrice initiale (n,m) avec les termes des deux vecteurs générateurs, en affectant à chaque zone élémentaire un terme de ligne et de colonne Bij représentant un coefficient d'atténuation et défini par la demi-somme, du terme homologue $c_i$ du vecteur générateur colonne divisé par le nombre m de termes du vecteur générateur ligne et du terme homologue $\rho_j$ du vecteur générateur ligne divisé par le nombre n de termes du vecteur générateur colonne,

$$B_{ij} = \frac{1}{2}\left(\frac{\rho_j}{n} + \frac{c_i}{m}\right)$$

(3) ajuster le coefficient d'atténuation en chaque zone élémentaire en utilisant la formule suivante :

$$C_{ij} = \frac{\rho_j}{n} + \frac{c_i}{m} - \frac{1}{2nm}\left(\sum_{i=1}^{n} c_i + \sum_{j=1}^{m} \rho_j\right)$$

où, dans cette formule,

Cij = la valeur recherchée du coefficient d'atténuation de la zone élémentaire (i,j) du quadrillage

Bij = la valeur estimée initialement

(n) = le nombre de lignes de la matrice initiale

(m) = le nombre de colonnes de la matrice initiale

$\rho_j$ est le j-ème terme du vecteur générateur de ligne

$c_i$ est le i-ème terme du vecteur générateur de colonne

pour aboutir à une image du plan de coupe du corps examiné sous les premier et deuxième angles de rotation, correspondant à une matrice ajustée pour laquelle les valeurs de bordure de ligne et de colonne calculées à l'aide des valeurs ajustées (Cij) sont égales, pour chaque ligne et pour chaque colonne, respectivement aux termes des vecteurs générateurs ligne et colonne,

$$\sum_{j=1}^{m} Cij = c_i$$

$$\sum_{i=1}^{n} Cij = \rho_j$$

(4) répéter les étapes (1) à (3) pour des données acquises avec différentes paires d'angles de rotation pour aboutir respectivement à différentes matrices ajustées correspondant à différentes images du plan de coupe du corps examiné sous les différentes paires d'angles de rotation,

(5) à l'aide d'un opérateur de rotation, superposer sur une même paire d'angles toutes les matrices ajustées, et

(6) afficher à l'écran de l'ordinateur une image de synthèse du plan de coupe du corps examiné correspondant à une matrice de synthèse des coefficients d'atténuation en chaque zone élémentaire (i,j) du quadrillage, obtenus par une moyenne terme à terme de toutes les matrices ajustées et superposées.

Sur la figure 10, le point considéré $X_N$ du détecteur 9 est par exemple constitué par la photodiode $x_N^k$ ayant le numéro k dans la dernière ligne du détecteur 9. Les données $c_i$ proviennent des intensités détectées par cette photodiode tout au long de la translation du détecteur 9, sous l'angle de rotation i du support 1 autour de l'axe de rotation. De même, les données $\rho_j$ proviennent des intensités détectées par cette photodiode tout au long de la translation du détecteur 9, sous l'angle de rotation j, décalé de 90 degrés vis-à-vis de l'angle de rotation i.

Lorsque, selon le deuxième mode de réalisation de l'invention, la photodiode $x_N^k$ est irradiée à la fois par des rayons réfléchis 17 par le réflecteur paraboloïde et des rayons directs 27 ou collimatés par le tube de collimation 24, les données $c_i$ ou $\rho_j$ provenant des intensités détectées par cette photodiode sous l'angle de rotation i ou j sont surestimées. Etant observé, qu'au cours de la rotation du support 1 autour de l'axe de rotation $\Omega$, une même zone élémentaire attachée au corps à examiner 3 n'est placée devant la photodiode $x_N^k$ que pour l'angle de rotation i ou j, la moyenne terme à terme effectuée à l'étape (6) du procédé tend à lisser l'effet de la surestimation. En fonction de l'écart-type de la moyenne obtenue, il est prévu d'effectuer un écrêtage des données surestimées.

Pour obtenir une image comportant (m x n) points du corps à examiner 3 dans le plan de coupe P, on construit, aux étapes (1) et (2), une matrice initiale de (m x n) termes par une estimation de chacun des termes des vecteurs générateurs colonne et ligne à partir des données obtenues à la suite seulement de deux irradiations du corps à examiner sous deux angles de rotation, par exemple décalés de 90 degrés.

A l'étape (3), on procède à un ajustement des termes estimés de la matrice initiale par rapport aux n et m valeurs moyennes d'intensité détectés en tenant compte des valeurs de bordure. On aboutit ainsi à une première matrice ajustée correspondant à une première image ajustée.

En pratique, les étapes (2) et (3) sont regroupées en une seule étape dite de génération directe des valeurs recherchées du coefficient d'atténuation Cij de l'image du plan de coupe du corps examiné sous les premier et deuxième angles de rotation. La formule utilisée à l'étape (3) ne nécessite en effet que la connaissance des données $c_i$ et $\rho_j$ provenant des intensités détectées dans la bande du détecteur pour le premier et le deuxième angle de rotation. Le troisième terme

$$\frac{1}{2nm}\left(\sum_{i=1}^{n} c_i + \sum_{j=1}^{m} \rho_j\right)$$

est constant et est calculé une seule fois pour toutes les valeurs recherchées Cij. Les opérations élémentaires mises en oeuvre par la formule conduisent à un gain de temps très important. De surcroît, les coefficients Cij sont calculés indépendamment d'une image à l'autre, en ce sens qu'aucune rétro- projection n'est effectuée entre deux images obtenues sous deux paires d'angles de rotation. L'image de synthèse est simplement le résultat d'une moyenne terme à terme des valeurs Cij des images obtenues pour les différentes paires d'angle. Cela conduit à une réduction très importante des erreurs de calcul.

L'exposé général ci-dessus s'applique à la scanographie d'un corps à examiner de petite taille selon les différentes variantes à foyer virtuel du premier mode de réalisation de l'invention décrites précédemment. Le support 1 est par exemple une éprouvette ou un tube à essai actionné en rotation autour de l'axe de rotation $\Omega$. Des résolutions d'image de 100 ou 25 angströms d'un tissu cellulaire ouvrent la voie à une exploration des éléments constitutifs des cellules, notamment à une comparaison entre le volume du noyau cellulaire et le volume du cytoplasme dans la perspective d'une caractérisation des cellulaires cancéreuses.

Les différentes prises de vue doivent être centrées sur un même repère. Dans le dispositif selon l'invention, où le détecteur est commandé en translation sur une largeur relativement importante, le centrage des prises de vue par rapport à la course du détecteur peut conduire à des imprécisions d'autant plus importantes que le poids du détecteur est plus élevé et que la vitesse de déplacement est plus importante. A noter que l'ensemble constitué par la tige mobile 28 du vérin 16 et le détecteur 9 présente une masse d'environ 15 kg. C'est la raison pour laquelle on préfère utiliser un système de centrage des prises de vue indépendant de la course du détecteur.

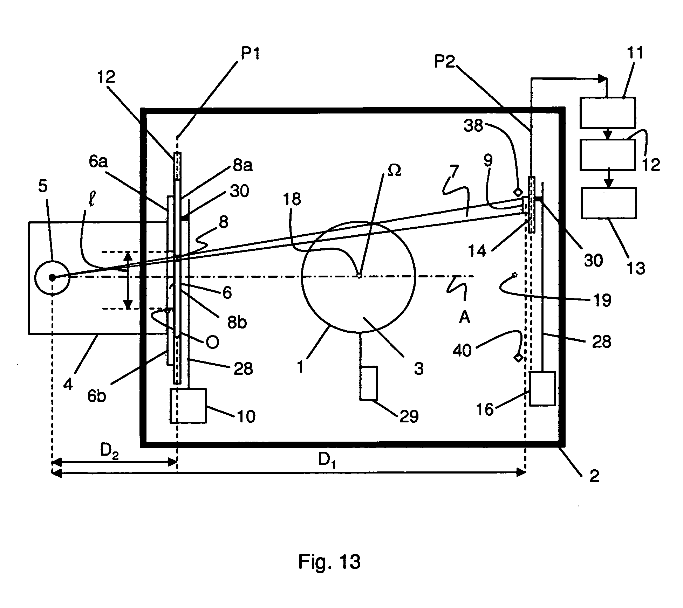

Figure 13, le système de centrage des prises de vue comprend un repère 38 fixe par rapport au détecteur 9 et un fil à plomb 18 suspendu au plafond de la cabine 2. Le repère fixe 38, par exemple une pointe, est disposé devant le plan de déplacement P2 du détecteur 9, dans une zone de hors champ par rapport à une projection du corps à examiner 3, c'est-à-dire excentrée par rapport à la direction axiale A du faisceau. Le fil à plomb 18 est disposé à l'aplomb du centre de rotation du support 1 en l'absence du corps à examiner pour matérialiser l'axe de rotation Ω. Figure 10, les projections 34 et 42 respectivement du fil à plomb 18 et du repère fixe 38 sont enregistrées lors d'une prise de vue de référence 47. La position de la projection du fil à plomb 18 est ensuite déterminée par rapport à la projection du repère fixe 38 et enregistrée en tant que position de la projection de l'axe de rotation Ω. Toute prise de vue effectuée après le retrait du fil à plomb 18 est ainsi recentrée sur l'axe de rotation Ω par rapport à la projection 42 du repère fixe 38.

On contrôle le parallélisme du détecteur 9 et de l'axe de rotation Ω, c'est-à-dire la verticalité du détecteur, par l'emploi d'un simple fil à plomb et corrige un éventuel défaut de parallélisme en agissant sur l'inclinaison du vérin 16. Il faut en effet garder présent à l'esprit que le vérin 16 peut subir, au cours des nombreuses manipulations, une légère inclinaison entraînant un défaut de verticalité du détecteur 9. Le système de centrage peut aussi comprendre un deuxième repère fixe 40, homologue du premier repère fixe 38, pour contrôler le parallélisme entre le détecteur 9 et l'axe de rotation Ω. Les deux repères 38 et 40 sont disposés à deux hauteurs différentes suivant l'axe de rotation Ω.

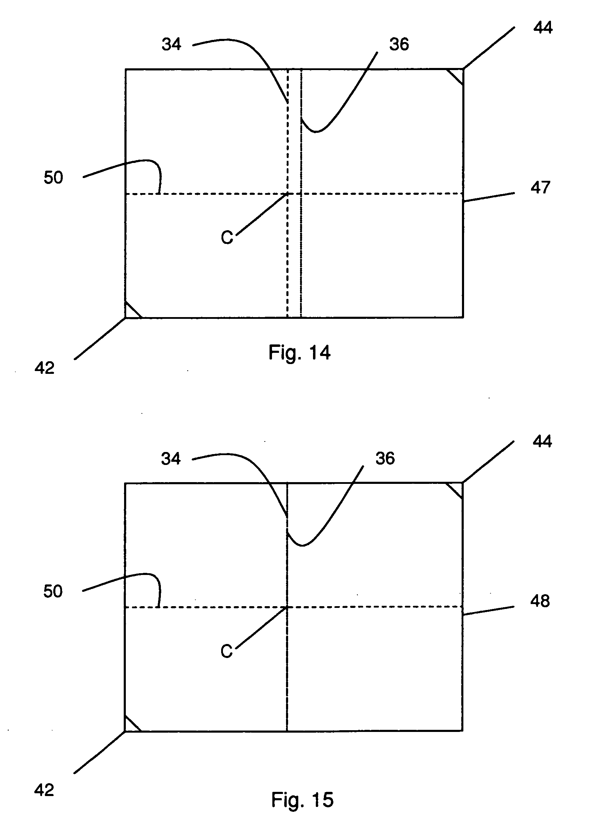

A noter que le système de centrage peut comprendre un deuxième fil à plomb 19 disposé devant le plan de déplacement P2 du détecteur, dans une position réglable. Avant centrage, il est disposé approximativement suivant l'alignement entre le foyer F de la source 5 et l'axe de rotation Ω matérialisé par le premier fil suspendu 18. La figure 14 montre la prise de vue de référence 47, où les projections 34 et 36 respectivement du premier fil 18, matérialisant l'axe de rotation Ω, et du deuxième fil 19 sont décalées l'une par rapport à l'autre. On règle la position de suspension du deuxième fil 19 au plafond de la cabine 2 de manière à le disposer exactement suivant l'alignement entre le foyer F de la source 5 et le premier fil suspendu 18. On contrôle cet l'alignement par une deuxième prise de vue de référence 48 où, figure 15, les projections 34 et 36 des deux fils suspendus 18 et 19 sont confondues.

Le deuxième fil à plomb 19 conserve la projection de l'axe de rotation Ω sur le détecteur et par conséquent sur toute prise de vue effectuée après le retrait du premier fil suspendu 18. Il permet de centrer les différentes prises de vue sur l'axe de rotation Ω matérialisé par la projection du deuxième fil suspendu 19.

Le premier fil 18 est par exemple en cuivre. Le deuxième fil 19 est de préférence plus absorbant aux rayons X que le premier. Il est par exemple en alliage d'or. Un tel alliage permet également de former un fil plus fin, par exemple de 0,6 mm de diamètre. Cependant, la présence du corps à examiner 3 peut dans certains cas masquer le deuxième fil à plomb 19 et c'est la raison pour laquelle on préfère utiliser le système de centrage par le repère fixe 38 disposé hors champ par rapport à la projection du corps à examiner 3.

Le dispositif selon l'invention peut également comprendre un moyen de centrage horizontal, c'est-à-dire perpendiculairement à l'axe de rotation Ω. Il peut s'agir d'une barrette disposée par exemple dans la cage parallélépipédique 4 ou dans le tube de collimation 24. Figures 10 et 11, la projection 50 de cette barrette sur les prises de vue de référence 47 et 48 est repérée par rapport à la projection 42 du repère fixe 38. L'intersection des projections 42 et 34 de la barrette et du fil à plomb 18 matérialisant l'axe de rotation Ω détermine le centre C de toute prise de vue, lui-même matérialisant la projection du foyer F de la source 5. A partir du centre C, il est permis de corriger les données issues de la conversion des intensités détectées pour tenir compte de la variation de distance entre le foyer F et une photodiode du détecteur 9 à tout instant de la translation de ce dernier. Il faut ici rappeler que l'intensité des rayons X décroît en raison inverse du carré de la distance de propagation.

Le dispositif d'imagerie X ou infrarouge selon l'invention permet d'obtenir des images à haute définition, multiple de 27 µm, tout en limitant au minimum l'exposition du patient et du personnel utilisant le dispositif aux rayons X ou infrarouges.

Des essais effectués ont conduit aux résultats suivants:

   i) en réglant l'ouverture de la fente du limiteur de dose à 2 cm, on obtient une dose moyenne de 1,725 milligrays sur le patient.

   ii) en réglant l'ouverture de la fente du limiteur de dose à 0,5 cm, on obtient une dose moyenne de 0,0983 milligrays sur le patient. Dans ce cas, des mesures effectuées autour de la cabine conduisent à des valeurs comparables à la radioactivité naturelle.

En ce qui concerne le patient, la dose reçue dépend de la puissance de la source nécessaire pour traverser la partie du corps à examiner avec les rayons X. On estime aujourd'hui :

   iii) pour une partie large, par exemple le thorax, la dose reçue pourra être de 0,5 milligrays avec une ouverture de fente égale à 0,5 cm.

   iv) pour une partie étroite, comme la main, la dose pourra être de seulement 0,090 milligrays avec une même ouverture de fente de 0,5 cm.

En fonction du nombre de prises de vue nécessaires à la formation de l'image de synthèse, la dose totale reçue par le patient pourra varier entre 1 et 6 milligrays. A noter que le réflecteur parabolique, selon le deuxième mode d'exécution de l'invention, permet de diminuer encore la dose reçue par le patient à même puissance de source.

Il importe également de souligner les faibles temps d'exposition aux rayons X permis par l'utilisation d'un dispositif selon l'invention. Pour une partie du corps large, comme le thorax, le temps d'acquisition d'une vue de 45 cm de largeur et de 30 cm de hauteur est égal à 5,55 secondes. Le temps total pour réaliser une scanographie à 18 vues atteint 190 secondes, en tenant compte des temps morts imposés par la rotation du support, ou atteint 380 secondes pour une scanographie à 36 vues.

Il faut encore mettre en relief les faibles temps de calcul pour le traitement des données, pour une cabine comprenant 4 systèmes source - limiteur de dose - détecteur totalisant 184 cm dans la hauteur et permettant d'obtenir la scanographie complète d'un patient en une seule opération :

v) 361 secondes à une définition de 162 $\mu$m, dans l'hypothèse où la translation du détecteur est la même, quelle que soit la partie du corps à examiner, conduisant à un important volume de données enregistrées pour des zones vides.

vi) 117 secondes à une définition de 81 $\mu$m, dans l'hypothèse où la translation de chacun des 4 détecteurs est ajutée à la largeur du corps à examiner explorée par ce détecteur.

Etant rappelé que le traitement des données permet de restituer une image de synthèse à toute définition multiple de la définition du détecteur, par exemple 27 $\mu$m, le dispositif d'imagerie selon l'invention offre à un médecin plusieurs protocoles, parmi lesquels :

- choisir la définition de la détection, 27 $\mu$m sur les N lignes du détecteur ou 81 $\mu$m sur le tiers de ces lignes regroupées par trois,

- choisir la définition de l'image de synthèse, multiple de la définition de la détection, soit 81 $\mu$m, 162 $\mu$m, 324 $\mu$m, 648 $\mu$m, en fonction de la taille de la partie explorée,

- zoomer sur une partie plus précise de l'image de synthèse en passant à une définition plus fine, par exemple de 324 $\mu$m à 81 $\mu$m,.

En définitive, le dispositif d'imagerie selon l'invention constitue une réponse à une imagerie à haute définition, de grande largeur, à faible dose d'irradiation, à faible puissance d'émission, à faible encombrement et à faible coût de fabrication.

**Revendications**

1. Dispositif d'imagerie X ou infrarouge, du type radiographie ou scanographie, comprenant :

- un support (1) pour recevoir un corps à examiner (3),
- une source (5) alimentant un foyer (F,F1,F2) dont est issu un faisceau (7,71,72) de rayons X ou lumineux traversant une fente (8) d'un limiteur de dose (8,8a,8b) commandé en translation pour balayer le corps à examiner (3),
- un détecteur (9) commandé en translation pour être irradié ou illuminé par le faisceau balayant le corps à examiner (3) et détecter une intensité atténuée en fonction d'une traversée des rayons X ou lumineux à travers le corps à examiner (3),
- un convertisseur (11) analogique-numérique pour convertir les intensités détectées en données pour déterminer une atténuation par le corps à examiner (3) des rayons X ou lumineux, et
- un ordinateur (13) programmé pour traiter les données provenant de la conversion des intensités détectées pour aboutir à une image exprimant l'atténuation des rayons X ou lumineux par le corps examiné (3),

**caractérisé en ce que** le limiteur de dose (8,8a,8b) est commandé en translation à une vitesse proportionnelle à celle du détecteur (9), contrôlée par le rapport des distances (D2/D1) entre d'une part (D1) le foyer (F,F1,F2) et le détecteur (9) et d'autre part, (D2) le foyer (F,F1,F2) et le limiteur de dose (8,8a,8b).

2. Dispositif d'imagerie X ou infrarouge selon la revendication 1, **caractérisé en ce que** le faisceau de rayons X ou lumineux est un faisceau secondaire (72), issu d'un foyer virtuel (F2) formé par un faisceau primaire (71), émis par un foyer réel (F1) alimenté par la source (5) et réfléchi par un réflecteur à double réflexion (55) disposé entre la source (5) et le limiteur de dose (8,8a,8b).

3. Dispositif d'imagerie X ou infrarouge selon la revendication 2, **caractérisé en ce que** le limiteur de dose (8,8a,8b) est disposé entre le foyer virtuel (F2) et le corps à examiner (3) et déplacé en translation dans le même sens de déplacement que celui du détecteur (9).

4. Dispositif d'imagerie X ou infrarouge selon la revendication 2, **caractérisé en ce que** le limiteur de dose (8,8a,8b) est disposé entre le réflecteur à double réflexion (55) et le foyer virtuel (F2) et déplacé en translation dans le sens de déplacement opposé à celui du détecteur (9).

5. Dispositif d'imagerie X ou infrarouge selon la revendication 2 ou 4, **caractérisé en ce que** le réflecteur à double réflexion comprend un réflecteur d'entrée (57) s'étendant suivant une direction axiale (E) parallèlement à laquelle les rayons X ou lumineux issus du foyer réel (F1) sont réfléchis et un réflecteur de sortie (59) s'étendant suivant une direction axiale (S), parallèle mais non co-axiale à la direction axiale (E) du réflecteur d'entrée (57), le long de laquelle le foyer virtuel (F2) est formé par convergence des rayons X ou lumineux réfléchis par le réflecteur de sortie (59).

6. Dispositif d'imagerie X ou infrarouge, du type radiographie ou scanographie, comprenant :

   - un support (1) pour recevoir un corps à examiner (3),
   - une source (5) émettant un faisceau (7) de rayons X ou lumineux traversant une fente (8) d'un limiteur de dose (8,8a,8b) commandé en translation pour balayer le corps à examiner (3),
   - un détecteur (9) commandé en translation pour être irradié ou illuminé par le faisceau balayant le corps à examiner (3) et détecter une intensité atténuée en fonction d'une traversée des rayons X ou lumineux à travers le corps à examiner (3),
   - un convertisseur (11) analogique-numérique pour convertir les intensités détectées en données pour déterminer une atténuation par le corps à examiner (3) des rayons X ou lumineux, et
   - un ordinateur (13) programmé pour traiter les données provenant de la conversion des intensités détectées pour aboutir à une image exprimant l'atténuation des rayons X ou lumineux par le corps examiné (3),

   **caractérisé en ce que** le limiteur de dose (8,8a,8b) est commandé en translation à la même vitesse que celle du détecteur (9) devant un réflecteur (15), irradiant ou illuminant le corps à examiner (3) en réfléchissant les rayons émis par la source (5) suivant des nappes parallèles.

7. Dispositif d'imagerie X ou infrarouge selon la revendication 6, **caractérisé en ce que** le réflecteur (15) comprend un collimateur (24) pour la collimation des rayons non réfléchis par le réflecteur (15) sur la fente (8) du limiteur de dose (8,8a,8b) lors de son déplacement devant le réflecteur (15).

8. Dispositif d'imagerie X ou infrarouge selon la revendication 1 ou 6, **caractérisé en ce que** le limiteur de dose (8,8a, 8b) comprend deux plaques (8a,8b) déplaçables l'une par rapport à l'autre par un moyen de réglage (21,23,25) de l'ouverture de la fente (8).

9. Dispositif d'imagerie X ou infrarouge selon la revendication 8, **caractérisé en ce que** les deux plaques (8a,8b) du limiteur de dose (8,8a,8b) sont pourvues d'un profil de fente (32) correspondant à un décalage entre deux barrettes de détection (9a,9b) formant le détecteur (9).

10. Dispositif d'imagerie X ou infrarouge selon la revendication 1 ou 6, **caractérisé en ce que** le limiteur de dose (8,8a, 8b) est commandé en translation à partir d'un point de repos (O) pour lequel la fente (8) est en dehors d'une section de sortie (6) d'une cage (4) contenant la source (5) ou en dehors d'une section de sortie (20,60) du réflecteur (15,55) pour obstruer le faisceau (7).

11. Dispositif d'imagerie X ou infrarouge selon la revendication 1 ou 6, **caractérisé en ce que** le limiteur de dose (8,8a, 8b) est commandé en déplacement, identiquement ou proportionnellement, à la vitesse du détecteur (9) égale au

terme $\dfrac{U}{N\tau}$ où U est la largeur utile du détecteur irradiée ou illuminée par le faisceau à travers la fente (8) du limiteur de dose (8,8a,8b), N est le nombre de lignes à K photodiodes du détecteur (9) et $\tau$ est le temps de transfert de charge des photodiodes d'une ligne à la ligne adjacente, cumulativement de la première ligne ($I_1$) à la dernière ligne ($I_N$) du détecteur (9), ce dernier (9) étant déplacé en sens contraire au sens du transfert de charge, soit en translation de la gauche vers la droite si la dernière ligne ($I_N$) marque un bord gauche du détecteur (9) et la première ligne ($I_1$), un bord droit du détecteur (9).

**12.** Dispositif d'imagerie X ou infrarouge selon la revendication 11, **caractérisé en ce qu'**il comprend une interface (12) entre le convertisseur (11) analogique-numérique et l'ordinateur (13) transférant les données issues de la conversion des charges cumulées sur chacune des K photodiodes de la dernière ligne ($I_N$) du détecteur (9), à une fréquence égale à l'inverse du temps $\tau$ de transfert de charge des photodiodes.

**13.** Dispositif d'imagerie X ou infrarouge selon la revendication 1 ou 6, **caractérisé en ce que** le support (1) est commandé en rotation autour de l'axe de rotation ($\Omega$) et l'ordinateur (13) est programmé pour effectuer les étapes suivantes :

(1) construire un vecteur générateur colonne et un vecteur générateur ligne dont les n et m termes sont respectivement constitués par les données ($c_i, \rho_j$) provenant de la conversion des intensités détectées en un même point ($X_N$) du détecteur (9) pendant la translation de ce dernier pour respectivement un premier (i) et un deuxième (j) angle de rotation, de préférence différents entre eux de 90 degrés, du support 1 autour de l'axe de rotation ($\Omega$), les données correspondant à un quadrillage en n x m zones élémentaires d'un plan de coupe du corps à examiner 3, perpendiculaire à l'axe de rotation ($\Omega$) et contenant le point considéré ($X_N$) du détecteur (9),
(2) construire une matrice initiale (n,m) avec les termes des deux vecteurs générateurs, en affectant à chaque zone élémentaire un terme de ligne et de colonne (Bij) représentant un coefficient d'atténuation et défini par la demi-somme, du terme homologue ($c_i$) du vecteur générateur colonne divisé par le nombre (m) de termes du vecteur générateur ligne et du terme homologue ($\rho_j$) du vecteur générateur ligne divisé par le nombre (n) de termes du vecteur générateur colonne,

$$B_{ij}^{'} = \frac{1}{2}\left(\frac{\rho_j}{n} + \frac{c_i}{m}\right)$$

(3) ajuster le coefficient d'atténuation en chaque zone élémentaire en utilisant la formule suivante :

$$C_{ij} = \frac{\rho_j}{n} + \frac{c_i}{m} - \frac{1}{2nm}\left(\sum_{i=1}^{n} c_i + \sum_{j=1}^{m} \rho_j\right)$$

où, dans cette formule,

Cij = la valeur recherchée du coefficient d'atténuation de la zone élémentaire (i,j) du quadrillage
Bij = la valeur estimée initialement
(n) = le nombre de lignes de la matrice initiale
(m) = le nombre de colonnes de la matrice initiale
$\rho_j$ est le j-ème terme du vecteur générateur de ligne
$c_i$ est le i-ème terme du vecteur générateur de colonne

pour aboutir à une image du plan de coupe du corps examiné sous les premier et deuxième angles de rotation, correspondant à une matrice ajustée pour laquelle les valeurs de bordure de ligne et de colonne calculées à l'aide des valeurs ajustées (Cij) sont égales, pour chaque ligne et pour chaque colonne, respectivement aux termes des vecteurs générateurs ligne et colonne,

$$\sum_{j=1}^{m} Cij = c_i$$

$$\sum_{i=1}^{n} Cij = \rho_j$$

(4) répéter les étapes (1) à (3) pour des données acquises avec différentes paires d'angles de rotation pour aboutir respectivement à différentes matrices ajustées correspondant à différentes images du plan de coupe du corps examiné sous les différentes paires d'angles de rotation,

(5) à l'aide d'un opérateur de rotation, superposer sur une même paire d'angles toutes les matrices ajustées, et

(6) afficher à l'écran de l'ordinateur une image de synthèse du plan de coupe du corps examiné correspondant à une matrice de synthèse des coefficients d'atténuation en chaque zone élémentaire (i,j) du quadrillage, obtenus par une moyenne terme à terme de toutes les matrices ajustées et superposées.

**14.** Dispositif d'imagerie X ou infrarouge selon la revendication 1, 6 ou 13, **caractérisé en ce qu'**il comprend un repère (38,40) fixe par rapport au détecteur (9) et disposé hors champ par rapport à une projection du corps à examiner (3) sur le détecteur (9) ou un fil à plomb (19) fixe par rapport au détecteur (9) et aligné avec la source (5) et l'axe de rotation ($\Omega$).

**15.** Programme d'ordinateur permettant de commander en translation un limiteur de dose (8,8a,8b) et un détecteur (9) dans un dispositif selon la revendication 1 ou 6 lorsqu'il est chargé dans un ordinateur (13).

**Claims**

**1.** X-ray or infrared imaging apparatus, for radiography or scanning, comprising:

- a support (1) to receive a body to be examined (3),
- a X-ray or light ray source (5) provided with a focus (F,F1,F2) for emitting a beam (7,71,72) passing through a slit (8) of a dose limiter (8,8a,8b) driven in translation to sweep the body to be examined (3),
- a detector (9) driven in translation to be irradiated or illuminated by the beam sweeping the body in order to detect an intensity attenuated according to the passage of the X-rays or light rays through the body to be examined (3),
- an analogue-to-digital converter (11) to convert the detected intensities into data enabling an attenuation of the X-rays or light rays by the body to be determined, and
- a programmed computer (13) to process the data resulting from the conversion of the detected intensities in order to obtain an image representing the attenuation of the X-rays or light rays by the examined body (3),

**characterised in that**:

- the dose limiter (8,8a,8b) is driven in translation with a speed proportional with the detector (9), controlled by the ratio (D2/D1) of the distances between, on the one hand (D1), the focus (F,F2,F2) and the detector (9) and, on the other hand (D2), the focus (F,F1,F2) and the dose limiter (8,8a,8b).

**2.** An X-ray or infrared imagery apparatus according to claim 1, wherein the X-ray or light ray beam is a secondary beam (72), resulting from a virtual focus (F2) formed by a primary beam (71) emitted by an actual focus (F1) supplied by the source and reflected by a double reflection reflector (55) laid out between the source (5) and the dose limiter (8,8a,8b).

**3.** An X-ray or infrared imagery apparatus according to claim 2, wherein the dose limiter (8,8a,8b) is laid out between the virtual focus (F2) and the body to be examined (3) and displaced in translation in the same direction as the detector (9).

4. An X-ray or infrared imagery apparatus according to claim 2, wherein the dose limiter (8,8a,8b) is laid out between the double reflection (55) reflector and the virtual focus (F2) and displaced in the direction opposed to the detector (9).

5. An X-ray or infrared imagery apparatus according to claim 2 or 4, wherein the double reflection reflector comprises:

   - an input reflector (57) which extends along a first axial direction (E) and reflects the X-rays or light rays emitted by the actual focus (F1) parallel to said axial direction and,
   - an output reflector (59) which extends along a second axial direction (S) parallel but non-coaxial with the first axial direction (E) of the input reflector (57), and

   wherein the X-rays or light rays reflected by the output reflector (59) converge at the virtual focus (F2) on the second axial direction.

6. X-ray or infrared imaging apparatus, for radiography or scanning, comprising:

   - a support (1) to receive a body to be examined (3),
   - a X-ray or light ray source (5) which emits a beam (7,71,72) passing through a slit (8) of a dose limiter (8,8a, 8b) driven in translation to sweep the body to be examined (3),
   - a detector (9) driven in translation to be irradiated or illuminated by the beam sweeping the body in order to detect an intensity attenuated according to the passage of the X-rays or light rays through the body to be examined (3),
   - an analogue-to-digital converter (11) to convert the detected intensities into data enabling an attenuation of the X-rays or light rays by the body to be determined, and
   - a programmed computer (13) to process the data resulting from the conversion of the detected intensities in order to obtain an image representing the attenuation of the X-rays or light rays by the examined body (3),

   **characterised in that**:

   - the dose limiter (8,8a,8b) is driven in translation at the same speed than the detector (9), in front of a reflector (15) which irradiates or illuminates the body to be examined (3) with the rays emitted by the source and reflected in parallel webs.

7. An X-ray or infrared imagery apparatus according to claim 6, wherein the reflector (15) comprises a collimator (24) which collimates the non reflected rays towards the dose limiter slit (8) when the dose limiter moves (8,8a,8b) in front of the reflector (15).

8. An X-ray or infrared imagery apparatus according to claim 1 or 6, wherein the dose limiter (8,8a,8b) comprises two plates (8a,8b) moved one compared to the other by an adjustment means (21,23,25) to adjust the slit width (8).

9. An X-ray or infrared imagery apparatus according to claim 8, wherein the two plates (8a,8b) of the dose limiter (8,8a, 8b) are provided with a slit profile (32) corresponding to a shift between two detection rods (9a,9b) of the detector (9).

10. An X-ray or infrared imagery apparatus according to claim 1 or 6, wherein the dose limiter (8,8a,8b) is driven in translation starting from a rest point (O) for which the slit (8) is outside the output section (6) of a parallelepipedic box (4) which houses the source (5) or outside a output section (20,60) of the receptor (15,55) to close the beam (7).

11. An X-ray or infrared imagery apparatus according to claim 1 or 6, wherein the dose limiter (8,8a,8b) is driven with a speed identical or proportional to the U detector speed $\dfrac{U}{N\tau}$ where U is the useful width of the detector irradiated or illuminated by the beam through the dose limiter slit (8,8a,8b), N is the number of lines of the detector (9), K is the number of photodiodes per line and $\tau$ is the photodiode charge transfer time of one line to the adjacent line, cumulatively from a first line ($I_1$) to a last line ($I_N$) of the detector (9), driven in opposite direction with respect to the charge transfer direction, that is to say in translation from the left to the right if the last line ($l_N$) marks a left edge of the detector (9) and the first line ($l_1$), a right edge of the detector (9).

12. An X-ray or infrared imagery apparatus according to claim 11, wherein it comprises an interface between the analog-to-digital converter and the computer to transfer, at a frequency equal to the inverse charge transfer time $\tau$, the data resulting from the conversion of the cumulated charges of every K photodiode of the last line $l_N$ of the detector.

13. An X-ray or infrared imagery apparatus according to claim 1 or 6, wherein the support is driven in rotation around the axis of rotation $\Omega$ and the computer is programmed to carry out the following steps:

(1) to build a column generating vector and a line generating vector whose n and m terms respectively consist in data ($c_i, \rho_j$) resulting from the conversion of detected intensities at a same point ($X_N$) of detector (9) during its translation and respectively for a first (i) and a second (j) angles of rotation, preferably orthogonal, of the support (1) around the axis of rotation ($\Omega$), said data corresponding to a grid of n x m elementary areas of the body sectional plane which is perpendicular to the axis of rotation ($\Omega$) and contains the considered point ($X_N$) of detector (9),
(2) to build an initial matrix (n, m) with the terms of the two generating vectors, by assigning to each elementary area a line and column term (Bij) which represents an attenuation coefficient and is equal to the half sum of the homologous term ($c_i$) of the column generating vector divided by the number (m) of terms of the line generating vector and the homologous term ($\rho_j$) of the line generating vector divided by number (n) of terms of the column generating vector,

$$B_{ij} = \frac{1}{2}\left(\frac{\rho_j}{n} + \frac{c_i}{m}\right)$$

(3) to adjust the attenuation coefficient of each elementary area by using the following formula:

$$C_{ij} = \frac{\rho_j}{n} + \frac{c_i}{m} - \frac{1}{2nm}\left(\sum_{i=1}^{n} c_i + \sum_{j=1}^{m} \rho_j\right)$$

where, in this formula,

Cij = the desired value of the attenuation coefficient of the elementary area (i, j) of the grid
Bij = the initially estimated value
(n) = the line number of the initial matrix
(m) = the column number of the initial matrix
$\rho_j$ is the j term of the line generating vector
$c_i$ is the i term of the column generating vector

to obtain an image of the body sectional plane for the first and second angles of rotation, corresponding to an adjusted matrix for which the line and column border values calculated using the adjusted values (Cij) are respectively equal, for every line and every column, to the terms of the line and column generating vectors:

$$\sum_{j=1}^{m} Cij = c_i$$

$$\sum_{i=1}^{n} Cij = \rho_j$$

(4) to repeat steps (1) to (3) with data acquired for different pairs of rotation angles to respectively obtain different adjusted matrixes corresponding to different images of the body sectional plane for the different pairs of angles of rotation,

(5) to use a rotation operator to superimpose all the adjusted matrixes on a same pair of angles of rotation, and

(6) to display on the screen of the computer a synthesis image of the body sectional plane corresponding to a synthesis matrix of the attenuation coefficients of every elementary areas (i, j) of the grid, obtained by a term to term average of all the adjusted and superimposed matrixes.

**14.** An X-ray or infrared imagery apparatus according to claim 1, 6 or 13, further comprising a mark (38,40) fixed with respect to the detector (9) and laid out outside a projection of the body (3) on the detector (9) or comprising a wire (19) fixed with respect to the detector (9) and aligned with the source (5) and the axis of rotation (Ω).

**15.** Computer program enabling for a dose limiter (8,8a,8b) and a detector (9) to be driven in translation in an apparatus according to claim 1 or 6 when it is loaded in a computer (13).

**Patentansprüche**

**1.** Röntgen -oder infrarot bildgebende Vorrichtung, für Röntgen oder Scannen, mit:

- einer Unterstützung (1), um einen Körper (3) zu unterstützen,
- einer Quelle (5) mit einem Fokus (F,F1,F2) um einen Röntgen- oder Lichtstrahl (7,71,72) auszustrahlen, der durch einen Schlitz (8) eines in Translation angetriebenen Dosis Begrenzer (8,8a,8b) überschreitet, um den Körper (3) durchzulaufen,
- einem Detektor (9), der in Translation angetrieben wird, um von den Röntgenstrahlen oder Lichtstrahlen bestrahlt oder beleuchtet zu werden und eine beim Durchgang der Röntgenstrahlen oder Lichtstrahlen durch den Körper geschwächte Intensität zu registrieren,
- einem Analog-Digital Konverter (11), das die registrierten Intensitäten in Daten konvertiert, um eine Schwächung der Röntgenstrahlen oder Lichtstrahlen beim Durchgang durch den Körper (3) zu bestimmen, und
- einem programmierten Computer (13), der die von der Konvertierung der registrierten Intensitäten kommenden Daten verarbeitet, um zu einem Bild zu führen, das die Schwächung der Röntgenstrahlen oder Lichtstrahlen beim Durchgang durch den Körper (3) darstellt,

**dadurch gekennzeichnet, dass**:

der Dosis Begrenzer mit einer Geschwindigkeit in Translation angetrieben wird, die im Verhältnis zu der Geschwindigkeit des Detektors (9) steht und die durch den Abstand Verhältnis zwischen einerseits, dem Fokus (F,F1,F2) und dem Detektor (9) und andererseits, dem Fokus (F,F1,F2) and dem Dosis Begrenzer (8,8a,8b) bestimmt wird.

**2.** Röntgen -oder infrarot bildgebende Vorrichtung nach Anspruch 1, **dadurch gekennzeichnet dass**, der Röntgen- oder Lichtstrahl ein zweiter Strahl (72) ist, der sich aus einem virtuellen Fokus (F2) ergibt, der von einem ersten Strahl (71) gebildet wird, der von einem materialen und durch die Quelle (5) betriebenen Fokus (F1) ausgestrahlt wird und von einem zwischen der Quelle (5) und dem Dosis Begrenzer (8,8a,8b) angeordneten doppelte Reflektion Reflektor (55) reflektiert wird.

**3.** Röntgen -oder infrarot bildgebende Vorrichtung nach Anspruch 2, **dadurch gekennzeichnet dass**, der Dosis Begrenzer (8,8a,8b) zwischen dem virtuellen Fokus (F2) und dem Körper (3) angeordnet ist und in die gleiche Richtung wie den Detektor (9) in Translation bewegt wird.

**4.** Röntgen -oder infrarot bildgebende Vorrichtung nach Anspruch 2, **dadurch gekennzeichnet dass**, der Dosis Begrenzer (8,8a,8b) zwischen dem doppelte Reflektion Reflektor (55) und dem virtuellen Fokus (F2) angeordnet ist und in die Gegenrichtung zu dem Detektor (9) in Translation bewegt wird.

**5.** Röntgen -oder infrarot bildgebende Vorrichtung nach Anspruch 2 oder 4, **dadurch gekennzeichnet dass**, der doppelte Reflektion Reflektor aufweist:

- einen Eingang Reflektor (57), der sich entlang eine erste axiale Richtung (E) erstreckt und parallel zu dieser axialen Richtung die von dem materialen Fokus (F1) ausgestrahlten Röntgen- oder Lichtstrahlen reflektiert und,
- einen Ausgang Reflektor (59), der sich entlang eine zweite axiale Richtung (S) erstreckt, die parallel aber nicht koaxial zu der ersten axialen Richtung (E) des Eingang Reflektors (57) ist und,
- die von dem Ausgang Reflektor (59) reflektierten Röntgen- oder Lichtstrahlen auf den virtuellen Fokus (F2) auf die zweite axiale Richtung konvergieren.

**6.** Röntgen -oder infrarot bildgebende Vorrichtung, für Röntgen oder Scannen, mit:

- einer Unterstützung (1), um einen Körper (3) zu unterstützen,
- einer Quelle (5) um einen Röntgen- oder Lichtstrahl (7,71,72) auszustrahlen, der durch einen Schlitz (8) eines in Translation angetriebenen Dosis Begrenzer (8,8a,8b) überschreitet, um den Körper (3) durchzulaufen,
- einem Detektor (9), der in Translation angetrieben wird, um von den Röntgenstrahlen oder Lichtstrahlen bestrahlt oder beleuchtet zu werden und eine beim Durchgang der Röntgenstrahlen oder Lichtstrahlen durch den Körper geschwächte Intensität zu registrieren,
- einem Analog-Digital Konverter (11), das die registrierten Intensitäten in Daten konvertiert, um eine Schwächung der Röntgenstrahlen oder Lichtstrahlen beim Durchgang durch den Körper (3) zu bestimmen, und
- einem programmierten Computer (13), der die von der Konvertierung der registrierten Intensitäten kommenden Daten verarbeitet, um zu einem Bild zu führen, das die Schwächung der Röntgenstrahlen oder Lichtstrahlen beim Durchgang durch den Körper (3) darstellt,

**dadurch gekennzeichnet, dass**:

der Dosis Begrenzer mit einer gleichen Geschwindigkeit wie den Detektor (9) in Translation und bevor einem Reflektor (15) angetrieben wird und der Körper (3) von den Strahlen ausgestrahlt oder beleuchtet wird, die von dem Reflektor (15) in parallelen Flächen reflektiert werden.

**7.** Röntgen -oder infrarot bildgebende Vorrichtung nach Anspruch 6, **dadurch gekennzeichnet dass**, der Reflektor (15) einen Kollimator (24) aufweist, der die nicht reflektierten Strahlen zu dem Schlitz (8) des Dosis Begrenzer (8,8a, 8b) kollimiert, als der Dosis Begrenzer sich vor dem Reflektor (15) bewegt.

**8.** Röntgen -oder infrarot bildgebende Vorrichtung nach Anspruch 1 oder 6, **dadurch gekennzeichnet dass**, der Dosis Begrenzer (8,8a,8b) zwei Platten (8a,8b) aufweist, die eine relativ zu der anderen durch ein Mittel (21,23,25) bewegt werden, das zur Anpassung der Schlitzesöffnung (8) dient.

**9.** Röntgen -oder infrarot bildgebende Vorrichtung nach Anspruch 8, **dadurch gekennzeichnet dass**, die zweite Platten (8a,8b) des Dosis Begrenzer (8,8a,8b) ein Schlitzesprofile (32) aufweisen, das einer Versetzung zwischen zwei Detektionsstangen (9a,9b) entspricht, die den Detektor (9) bilden.

**10.** Röntgen -oder infrarot bildgebende Vorrichtung nach Anspruch 1 oder 6, **dadurch gekennzeichnet dass**, der Dosis Begrenzer (8,8a,8b) aus einem Ruhepunkt (O) in Translation angetrieben wird, wofür der Schlitz (8) sich außerhalb eines Ausgang Abschnittes (6) eines Kasten (4), der die Quelle (5) enthält, oder außerhalb eines Ausgang Abschnittes (20,60) des Reflektor (15,55) befindet, um den Strahl (7) zu versperren.

**11.** Röntgen -oder infrarot bildgebende Vorrichtung nach Anspruch 1 oder 6, **dadurch gekennzeichnet dass**, der Dosis Begrenzer (8,8a,8b) gleichmäßig order verhältnismäßig mit der Geschwindigkeit $\dfrac{U}{N\tau}$ des Detektors (9)

angetrieben wird, wo U für die aktive Breite des von dem Strahl durch den Schlitz (8) des Dosis Begrenzer (8,8a, 8b) ausgestrahlten oder Beleuchteten Detektors gilt, N für den Anzahl der Reihen des Detektors (9) gilt, jede mit K Photodioden, und $\tau$ für die Ladungsübertragungszeit der Photodioden gilt, aus einer Reihe zu der folgenden Reihe, zusammenzählend aus der ersten Reihe ($I_1$) zu der letzten Reihe ($I_N$) des Detektors (9), der gegenwärtig der Ladungsübertragungsrichtung in Translation angetrieben wird, das heißt, von links nach rechts als die letzte Reihe

($I_N$), beziehungsweise die erste Reihe ($I_1$), sich an einem rechten Rand, beziehungsweise an einem linken Rand, des Detektors (9) befindet.

12. Röntgen -oder infrarot bildgebende Vorrichtung nach Anspruch 11, **dadurch gekennzeichnet dass**, sie zwischen dem Analog-digital Konverter und dem Computer eine Interface umfasst, die zur Übertragung der Daten dient, die von der Konvertierung der auf jene der K Photodioden der letzte Reihe ($I_N$) des Detektors (9) zusammenzählenden Ladungen kommen, mit einer Frequenz die der Umkehr der Ladungsübertragungszeit τ der Photodioden gleich ist.

13. Röntgen -oder infrarot bildgebende Vorrichtung nach Anspruch 1 oder 6, **dadurch gekennzeichnet dass**, die Unterstützung (1) bezüglich auf die Drehachse (Ω) in Drehung angetrieben wird und der Computer (13) programmiert ist, um die folgende Schritte durchzuführen:

(1) ein Spalte erzeugender Vektor und ein Linie erzeugender Vektor zu erstellen, deren n und m Koordinaten beziehungsweise aus ($c_i$, $\rho_j$) Daten bestehen, die, als der Detektor in Translation angetrieben wird, von der Konvertierung der an einem gleichen Punkt ($X_N$) des Detektors (9) detektierten Intensitäten kommen, beziehungsweise für einen ersten (i) und einen zweiten (j) Rotationswinkel, vorzugsweise orthogonal zueinander, der Unterstützung (1) bezüglich auf die Drehachse (Ω), wobei die Daten einem Rasterfeld von n x m grundlegenden Bereichen einer Schnittfläche (P) des Körpers entsprechen, die senkrecht auf der Drehachse (Ω) gerichtet ist und den gennante Punkt ($X_N$) des Detektors (9) enthält,

(2) eine initiale Matrix (n, m) mit den Koordinaten der zwei erzeugenden Vektoren so zu erstellen, dass jedem grundlegenden Bereich eine Linie und Spalte Koordinate ($B_{ij}$) entspricht, die einen Schwächungskoeffizient darstellt und der halben Summe der homologen Koordinate ($c_i$) des Spalte Vektors, geteilt durch die Zahl (m) der Linie Vektor Koordinaten und der homologen Koordinate ($\rho_j$) des Linie Vektors, geteilt durch die Zahl (n) der Spalte Vektor Koordinaten, gleich ist,

$$B_{ij} = \frac{1}{2}\left(\frac{\rho_j}{n} + \frac{c_i}{m}\right)$$

(3) den Schwächungskoeffizient jedes grundlegenden Bereichs durch die Verwendung der folgenden Formel anzugleichen:

$$C_{ij} = \frac{\rho_j}{n} + \frac{c_i}{m} - \frac{1}{2nm}\left(\sum_{i=1}^{n} c_i + \sum_{j=1}^{m} \rho_j\right)$$

wo, in dieser Formel,

$C_{ij}$ = der gewünschte Wert des Schwächungskoeffizienten des grundlegenden Bereichs (i, j) des Rasterfeldes
$B_{ij}$ = der initiale abgeschätzte Wert
(n) = die Linie Zahl der initialen Matrix
(m) = die Spalte Zahl der initialen Matrix
$\rho_j$ ist die j-Koordinate des Linie erzeugenden Vektors
$c_i$ ist die i-Koordinate des Spalte erzeugenden Vektors,

um zu einem Bild der Schnittfläche (P) des Körpers für die ersten und zweiten Rotationswinkel zu führen, das einer angeglichenen Matrix entspricht, in derer die mit den angeglichenen Werten ($C_{ij}$) berechneten Linie und Spalte Randwerte, beziehungsweise den Koordinaten der Linie und Spalte erzeugenden Vektoren für jede Linie und jede Spalte gleich sind:

$$\sum_{j=1}^{m} Cij = c_i$$

$$\sum_{i=1}^{n} Cij = \rho_j$$

(4) Schritte (1) bis (3) mit den Daten zu wiederholen, die für unterschiedliche Paare Rotationswinkeln ermittelt werden, um beziehungsweise zu unterschiedlichen angeglichenen Matrizen zu führen, die unterschiedlichen Bildern der Körperschnittfläche für die unterschiedlichen Paare Rotationswinkeln entsprechen,
(5) alle angeglichenen Matrizen auf ein gleiches Paar Rotationswinkeln übereinander zu legen, mittels eines Rotationsoperators, und
(6) ein Synthesebild der Körperschnittfläche auf dem Schirm des Computers anzuzeigen, das einer Synthesematrix der Schwächungskoeffizienten jedes grundlegenden Bereiches (i, j) des Rasterfeldes entspricht, die durch eine Punkt für Punkt Durchschnittberechnung aller angeglichenen und übereinandergelegten Matrizen ermittelt sind.

14. Röntgen -oder infrarot bildgebende Vorrichtung nach Anspruch 1, 6 oder 13, **dadurch gekennzeichnet dass**, sie eine Markierung umfasst, die bezüglich auf den Detektor (9) unbeweglich ist und sich bezüglich auf eine Projektion des Körpers auf den Detektor (9) außer Feld befindet oder dass, sie ein Lot (19) umfasst, das bezüglich auf den Detektor (9) unbeweglich ist und mit der Quelle (5) und der Drehachse ($\Omega$) aufgereiht ist.

15. Computer Programm um einen Dosis Begrenzer (8,8a,8b) und einen Detektor (9) in Translation anzutreiben wenn es in einem Computer (13) eingeladen worden ist.

Fig. 1

Fig. 2

Fig. 3

Fig. 4

Fig. 5

Fig. 6

Fig. 7

Fig. 8

Fig. 9

Fig. 10

Fig. 11

Fig. 12

Fig. 13

Fig. 14

Fig. 15

## RÉFÉRENCES CITÉES DANS LA DESCRIPTION

**Documents brevets cités dans la description**

- FR 2888374 A
- EP 1058322 A
- US 6429572 B
- AT 71957 M
- FR 2888374
- WO 2007006560 A
- FR 2871911
- WO 2006003312 A